(19) Europäisches Patentamt
European Patent Office
Office européen des brevets

(11) EP 1 221 448 A1

(12) **EUROPEAN PATENT APPLICATION**
published in accordance with Art. 158(3) EPC

(43) Date of publication:
**10.07.2002 Bulletin 2002/28**

(21) Application number: **00961239.1**

(22) Date of filing: **26.09.2000**

(51) Int Cl.[7]: **C07K 5/075**, A23L 1/236, A23L 1/22, A23L 2/60

(86) International application number:
**PCT/JP00/06628**

(87) International publication number:
**WO 01/25262 (12.04.2001 Gazette 2001/15)**

(84) Designated Contracting States:
**AT BE CH CY DE DK ES FI FR GB GR IE IT LI LU MC NL PT SE**
Designated Extension States:
**AL LT LV MK RO SI**

(30) Priority: **04.10.1999 JP 28350599**
**04.10.1999 JP 28350699**
**05.10.1999 JP 28434699**

(71) Applicant: **Ajinomoto Co., Inc.**
**Tokyo 104-8315 (JP)**

(72) Inventor: **ISHII, Shoichi, Food Res. & Devel. Lab.**
**Kawasaki-shi, Kanagawa-ken 210-0801 (JP)**

(74) Representative: **Nash, David Allan et al**
**Haseltine Lake & Co.,**
**Imperial House, 15-19 Kingsway**
**London WC2B 6UD (GB)**

(54) **SWEETENER COMPOSITIONS WITH HIGH DEGREE OF SWEETNESS HAVING IMPROVED SWEETNESS, CORRIGENTS AND UTILIZATION THEREOF**

(57) A sweetener composition with a high intense sweetness having a sweetness quality which is closer to that of sucrose, obtained by blending aspartyl dipeptide ester derivatives which are sweetener components with a high intense sweetness such as N-[N-[3-(3-hydroxy-4-methoxyphenyl) propyl]-L-α-aspartyl]-L-phenylalanine 1-methyl ester, with anther sweetener components with a high intense sweetness such as Aspartame, or at least one of the compounds contained in the group consisting of sugar, sugar alcohol and oligosaccharides so as to strengthen the onset of the taste of said derivatives and weaken the lingering taste, thereby improving the balance of the onset of the taste and the lingering taste, is provided.

Further, said aspartyl dipeptide ester derivatives can be used as taste modifiers. By using these taste modifiers, an effect of highly eliminating or repressing a bitter taste can be achieved and maintained, without undesirable effect for the physical properties of compositions such as food and drink and medicines, in particular, viscosity and so on of liquid compositions, or degradation of qualities such as browning during storage. Thus, food and drink and medicines and so on of a preferable taste quality with the improved bitter taste can be provided.

EP 1 221 448 A1

## Description

### Technical Field

The present invention relates to a novel sweetener composition, more specifically, a sweetener composition with a high intense sweetness which comprises the specific aspartyl dipeptide ester derivative, for example, N-[N-[3-(3-hydroxy-4-methoxyphenyl) propyl]-L-α-aspartyl]-L-phenylalanine 1-methyl ester and an another sweetener with a high intense sweetness, for example, Aspartame, or at least one of the compounds contained in the group consisting of sugar, sugar alcohol and oligosaccharide, such as sucrose, wherein the sweetness quality from said derivative is improved, and a sweetener, food and drink or another sweetened product comprising the above sweetener composition, and further a method of imparting a sweet taste by using the above sweetener composition and so on.

In addition, the present invention relates to a novel taste modifier, more specifically, a.taste modifier comprising the specific aspartyl dipeptide ester derivative, for example, N-[N-[3-(3-hydroxy-4-methoxyphenyl) propyl]-L-α-aspartyl] -L-phenylalanine 1-methyl ester (which is referred to as "derivative 1"), and food and drink, medicine and so on, with improved bitter taste by using the above taste modifier and further a method for correcting the taste.

### Background Art

It has been reported that the sweetness intensity of Neotame which is a sweetener with a high intense sweetness is 10000 times that of sucrose by weight (refer to Japanese Patent Kohyo Publication JP-A-8-503206), and the sweetness intensity of Aspartame is 200 times that of sucrose by weight (refer to Japanese Patent Kokoku Publication JP-B-47-31031). These sweeteners have been commercially used already, or their research for the application is going on. Besides, although many other sweeteners with a high intense sweetness have been proposed, there are many problems for practical use.

It is still desired to develop a sweetener substance with a high intense sweetness which is different from the sweetness of conventional sweetener, and preferably a sweetener substance with a high intense sweetness excellent in a sweetness quality and a physical property such as stability and so on, or a highly practical sweetener composition with a high intense sweetness which comprises said sweetener substance.

On the other hand, with regard to a taste modifier, there has been performed many attempts to develop the method of removing and suppressing the bitter taste from bitter substances and products containing them. Particularly, in the field of medical supplies, there are many medicines which contain bitter taste in their effective ingredients. Thus, it is an important problem in the art of pharmaceutical preparation, to remove and suppress the bitter taste and to maintain the effect for a long time (refer to Japanese Patent Kokai Publication JP-A-6-2-98668).

With regard to the methods of removing and suppressing the bitter taste, sugar coating, use of coating materials, capsulation and use of inclusion compound are applied to the solid agent. As for a liquid agent, due to the difficulty of stable coating in the liquid compared to the solid agent, it has been applied to add high concentration of sugar or organic compound, and flavour and so on. Recently, it has been proposed to suppress the bitter taste by adding lecithin which has a function of suppressing a bitter taste.

However, there are several problems such that the suppression of the bitter taste is not sufficient by these methods and the effect is weakened by decomposing the ingredient during the period of storage in a solution. In particular, the addition of sugar is not suitable for the calorie-controlled patient such as diabetes due to its high concentration.

Moreover, for babies and children, it is difficult to take a tablet and a granular preparation, and there are often used the liquid agent and the dry syrup which is dissolved in use. Therefore it is a large problem to remove or suppress the bitter taste in the aqueous solution.

On the other hand, in case of food, there are many problems in various form of food, such as the bitter taste of peptides and amino acids containing hydrolyzed vegetable or animal protein, the bitter taste of fruit juice and so on, the bitter and astringent taste of minerals and so on which are added for enrichment of nutrition. It is employed for suppressing the bitter taste in food and drink to add a sweetener and an organic acid, to use an adsorbent and an inclusion compound, and to treat by enzyme and so on. Also proposed the method of adding lecithin as the case of medicine.

However, even if the above methods are used, it is often difficult to obtain the sufficient effect depending on the substance of bitter taste.

In addition, it is not appropriate for the calorie-controlled patient such as diabetes to add the sweetener, e.g. sugar. On the other hand, although the addition of the sweetener with a high intense sweetness has no problem for carblie-controlling due to its little amount of addition, it cannot suppress the bitter taste of the product by generating the intrinsic bitter taste, for example in case of Glycyrrhizin. When it is used in the aqueous solution, there is further problem to reduce the effect by decomposing in the aqueous solution.

## Problem to be solved by the Invention

As a result of research to develop a sweet substance with a high intense sweetness, it was found that the aspartyl dipeptide ester derivative represented by the following general formula (2) had a high intense sweetness and was useful for a sweetener with a high intense sweetness. The patent application of these contents (inventions) had been already filed by the present applicant.

According to a further study by the present inventor, although the magnification of intense sweetness of said derivative is extremely high, the onset of the taste (the early taste) of said derivative is very weak compared to that of sucrose, and the lingering taste (the later taste) is felt very strongly. Therefore, it has been found to be necessary to develop a sweetener composition with a high intense sweetness having a good and well-balanced sweetness quality for preparing a sweetener with superior sweetness quality.

Thus, it is a problem to be solved by, or an object of the present invention to provide a sweetener composition having a superior sweetness quality by approximating the balance and so on of the onset of the taste (which means that a sweetener when put in the mouth tastes sweet as early as sucrose) and the lingering taste (which means that a sweetener when put in the mouth tastes sweet later than sucrose) of said derivative, to the sweetness quality of sucrose much more.

On the other hand, with regard to the taste modifier and the method of correcting a taste, there are many problems in the previously known and proposed methods. Thus, it is desired to develop a taste modifier which can be widely applied to food and drink and medicine and so on hereafter and in particular can be obtained a superior effect in the aqueous solution.

It is a further object of the present invention to develop a taste modifier which can remove the bitter taste, exhibit the effect of suppressing the bitter taste, and maintain the effect for a long time, without undesirable effect for the physical property of the composition, especially, the property of liquid composition such as viscosity, and degradation of quality such as browning during the storage.

## Disclosure of the Invention

The present inventor has studied eagerly to solve the problem described above and have found that a well-balanced sweetener composition, can be obtained by combining said derivative with a high intense sweetness and another sweetener with a high intense sweetness to make the onset of the taste stronger and on the other hand the lingering taste weaker. This finding led to the accomplishment of one of the present invention which is referred to as "the first invention of the present invention" or "The present invention (the first invention)".

As a result of further study by the present inventor, they found that the sweetener composition which has a well-balanced sweetness quality is obtained by combining said derivatives with a high intense sweetness and at least one of the compounds contained in the group consisting of sugar, sugar alcohol and oligosaccharide, to be strong in the onset of the taste and contrary weak in the lingering taste, and these findings have led to the another embodiment of the invention. This invention (another embodiment) is referred to as " the second invention of the present invention" or "the present invention (the second invention)".

Next, the present inventor has studied eagerly to solve the problem concerning the taste modifier described above and have found that the following derivatives with a high intense sweetness can remove or suppress the bitter taste, and maintain the effect for a long time, without undesirable effect for the physical property of the compositions by using together with the substance of bitter taste or food and drink, medicines and so on which contain the substance of bitter taste, and exert the bitter taste, and these findings have led to another embodiment of the invention. This invention (further another embodiment) is referred to as " the third invention of the present invention" or "the present invention (the third invention)".

Accordingly, the present invention relates to a novel sweetener composition with a high intense sweetness having an improved sweetness, a novel taste modifier and uses thereof, and includes three inventions of the first, second and third inventions, each belonging to one embodiment of the present invention.

(The first invention of the present invention)

The present invention (the first invention) lies in a sweetener composition with a high intense sweetness comprising an aspartyl dipeptide ester derivative (which may be in the salt form) represented by the following general formula (2), more preferably the following general formula (1), and an another sweetener with a high intense sweetness, wherein the sweetness quality from said derivative is improved.

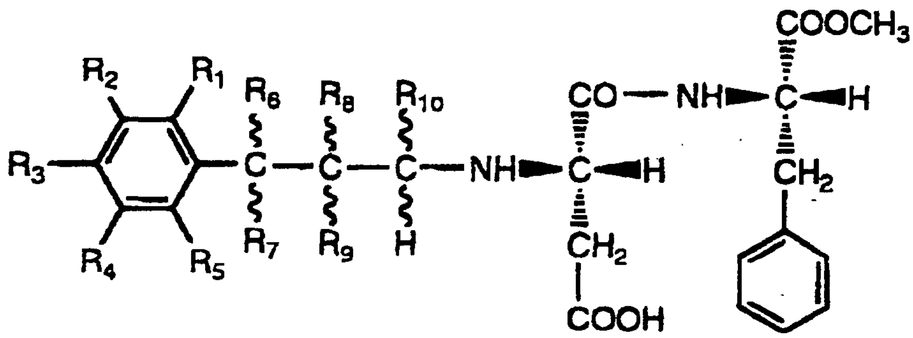

(2)

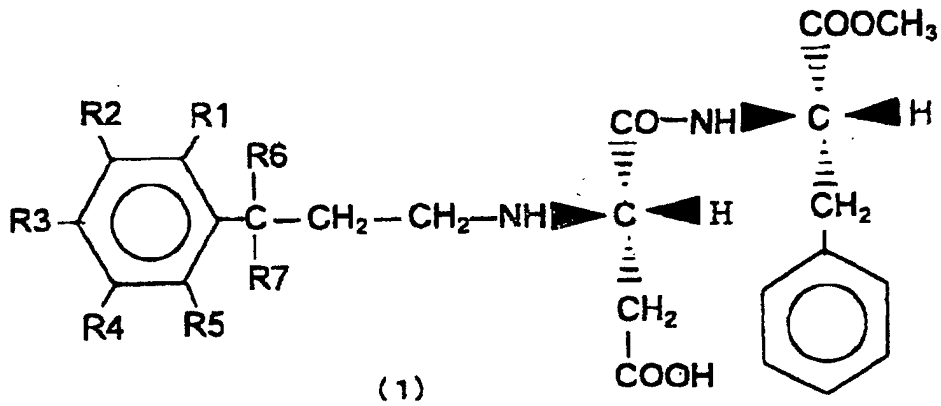

(1)

wherein in the above formulae, $R_1$, $R_2$, $R_3$, $R_4$ and $R_5$ are independent from each other, and each denotes any one selected from the group consisting of a hydrogen atom, a hydroxyl group, an alkoxy group having 1 to 3 carbon atoms (methoxy, ethoxy, n-propoxy and so on), an alkyl group having 1 to 3 carbon atoms (methyl, ethyl, n-propyl and so on) and a hydroxyalkyloxy group having 2 or 3 carbon atoms ($O(CH_2)_2OH$, $OCH_2CH(OH)CH_3$ and so on), and herein as for $R_1$ and $R_2$, or $R_2$ and $R_3$, the respective two symbols ($R_1$ and $R_2$, or $R_2$ and $R_3$), combined together with each other, may form a methylene dioxy group ($OCH_2O$).

$R_6$, $R_7$, $R_8$, $R_9$ and $R_{10}$ are independent from each other, and each denotes a hydrogen atom or an alkyl group having 1 to 3 carbon atoms (methyl, ethyl, isopropyl and so on), respectively, herein any two substituents optionally selected from the group consisting of $R_6$, $R_7$, $R_8$, $R_9$ and $R_{10}$, combined together with each other, may form an alkylene group having 1 to 5 carbon atoms ($CH_2$, $CH_2CH_2$, $CH_2CH_2CH_2$ and so on).

When $R_6$ and $R_7$, or $R_8$ and $R_9$ denote different substituents with each other, or $R_{10}$ denotes an substituent except for a hydrogen atom, the configuration of the carbon atom to which these substituents ($R_6$ and $R_7$, $R_8$ and $R_9$ or $R_{10}$) are linked, has no restriction, and may be any one of (R), (S) and (RS) or mixture thereof, for example. In addition, wiggly lines described as the bond of $R_6$ to $R_{10}$, and an hydrogen atom with a carbon atom in said general formula (2), mean that the direction of the bond is free (is not specified).

However, the derivative in which $R_6$ denotes a hydrogen atom or a methyl group and $R_1$, $R_2$, $R_3$, $R_4$, $R_5$, $R_7$, $R_8$, $R_9$ and $R_{10}$ denote a hydrogen atom concurrently is excluded. The derivative in which $R_2$ or $R_4$ denote a methoxy group, $R_3$ denotes a hydroxyl group, $R_{10}$ denotes a hydrogen atom or a methyl group, and $R_1$, $R_4$, $R_5$, $R_6$, $R_7$, $R_8$, and $R_9$ denote a hydrogen atom concurrently is also excluded.

As an aspartyl dipeptide ester derivative used for the present invention (the first invention), the derivative represented by said formula wherein all of $R_8$, $R_9$ and $R_{10}$ denote a hydrogen atom is more preferable.

The sweetness intensity of the aspartyl dipeptide ester derivative used for the present invention (the first invention) is preferably more than 4,000 times that of sucrose.

The following contents are also included in the aspartyl dipeptide ester derivatives used for the present invention (the first invention) as a preferable derivative thereof.

[1] The derivative of the formula (2) described above, wherein $R_3$ is a hydroxyl group or a methoxy group, and $R_4$ and $R_5$ are a hydrogen atom.

[2] The derivative of the formula (2) described above, wherein $R_1$ is a hydroxyl group.
[3] The derivative of the formula (2) described above, wherein $R_1$ is a hydrogen atom.
[4] The derivative of the formula (2) described above, wherein $R_2$, $R_6$ and $R_7$ are a hydrogen atom.
[5] The above any derivative of the formula described above,

wherein $R_2$ is a hydrogen atom, a hydroxyl group or a methyl group.
The derivatives described above include those in the form of salts, for example, edible salts form such as hydrochloride salts, sodium salts, potassium salts, ammonium salts, calcium salts and magnesium salts and so on.
For the particularly preferable aspartyl dipeptide ester derivative used for the present invention (the first invention), 9 derivatives shown in the following table 1 can be enumerated. (All of $R_8$, $R_9$ and $R_{10}$ denote a hydrogen atom.)

[Table 1]

| Derivative No. | $R_1$ | $R_2$ | $R_3$ | $R_4$ | $R_5$ | $R_6$ | $R_7$ |
|---|---|---|---|---|---|---|---|
| 1 | H | OH | $OCH_3$ | H | H | H | H |
| 2 | H | H | $OCH_3$ | H | H | H | H |
| 3 | H | OH | $OCH_3$ | H | H | $CH_3$ | $CH_3$ |
| 4 | H | $CH_3$ | OH | H | H | $CH_3$ | $CH_3$ |
| 5 | H | H | $OCH_3$ | H | H | $CH_3$ | $CH_3$ |
| 6 | H | H | OH | H | H | $CH_3$ | $CH_3$ |
| 7 | OH | H | $OCH_3$ | H | H | H | H |
| 8 | H | $CH_3$ | OH | H | H | H | H |
| 9 | OH | H | OH | H | H | H | H |

A sweetener, food and drink and other sweetened product and so on which comprise such a sweetener composition, are also contained in the present invention.

(The second invention of the present invention)

The present invention (the second invention) lies in a sweetener composition comprising an aspartyl dipeptide ester derivative (which may be in the salt form) represented by the following general formula (2), more preferably the following general formula (1), and at least one of the compounds contained in the group consisting of sugar, sugar alcohol and oligosaccharide, wherein the sweetness quality from said derivative is improved. Said aspartyl dipeptide ester derivatives used for the present invention (the second invention) includes those in the form of salts, and only one derivative and the mixture of not less than two derivatives are included therein.

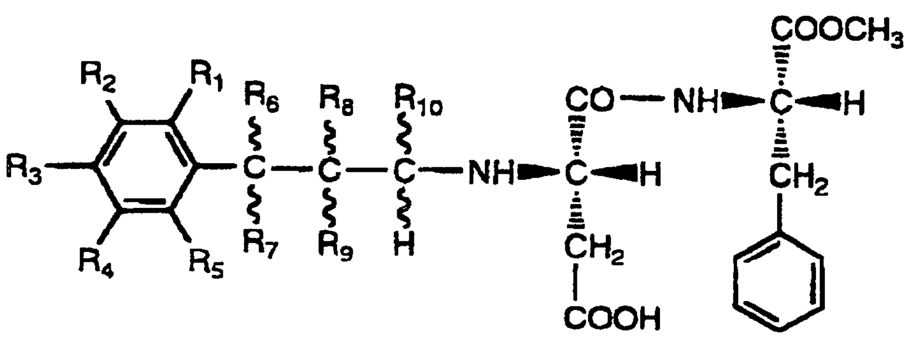

(2)

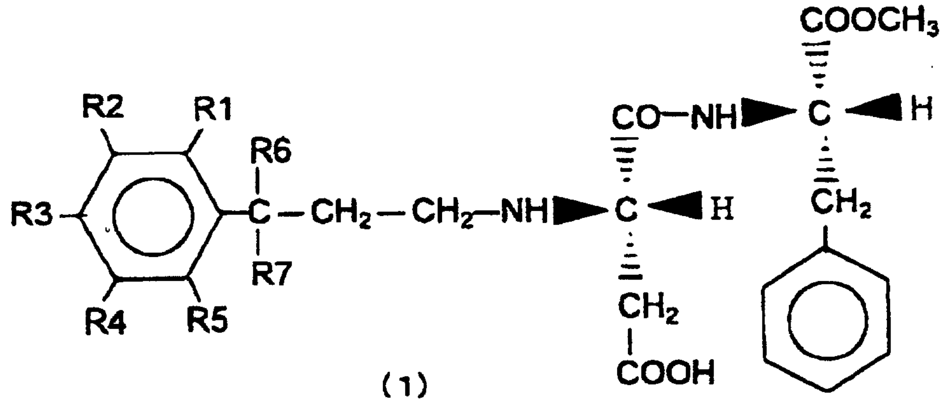

(1)

wherein in the above formulae, $R_1$, $R_2$, $R_3$, $R_4$ and $R_5$ are independent from each other, and each denotes any one selected from the group consisting of a hydrogen atom, a hydroxyl group, an alkoxy group having 1 to 3 carbon atoms (methoxy, ethoxy, n-propoxy and so on), an alkyl group having 1 to 3 carbon atoms (methyl, ethyl, n-propyl and so on) and a hydroxyalkyloxy group having 2 or 3 carbon atoms (O $(CH_2)_2OH$, $OCH_2CH(OH)CH_3$ and so on), and herein as for $R_1$ and $R_2$, or $R_2$ and $R_3$, the respective two symbols ($R_1$ and $R_2$, or $R_2$ and $R_3$), combined together with each other, may form a methylene dioxy group ($OCH_2O$).

$R_6$, $R_7$, $R_8$, $R_9$ and $R_{10}$ are independent from each other, and each denotes a hydrogen atom or an alkyl group having 1 to 3 carbon atoms (methyl, ethyl, isopropyl and so on), respectively, herein any two substituents optionally selected from the group consisting of $R_6$, $R_7$, $R_8$, $R_9$ and $R_{10}$, combined together with each other, may form an alkylene group having 1 to 5 carbon atoms ($CH_2$, $CH_2CH_2$, $CH_2CH_2CH_2$ and so on).

When $R_6$ and $R_7$, or $R_8$ and $R_9$ denote different substituents with each other, or $R_{10}$ denotes an substituent except for a hydrogen atom, the configuration of the carbon atom to which these substituents ($R_6$ and $R_7$, $R_8$ and $R_9$ or $R_{10}$) are linked, has no restriction, and may be any one of (R), (S) and (RS) or mixture thereof, for example. In addition, wiggly lines described as the bond of $R_6$ to $R_{10}$, and an hydrogen atom with a carbon atom in said general formula (2), mean that the direction of the bond is free (is not specified).

However, the derivative in which $R_6$ denotes a hydrogen atom or a methyl group and $R_1$, $R_2$, $R_3$, $R_4$, $R_5$, $R_7$, $R_8$, $R_9$ and $R_{10}$ denote a hydrogen atom concurrently is excluded. The derivative in which $R_2$ or $R_4$ denote a methoxy group, $R_3$ denotes a hydroxyl group, $R_{10}$ denotes a hydrogen atom or a methyl group, and $R_1$, $R_4$, $R_5$, $R_6$, $R_7$, $R_8$, and $R_9$ denote a hydrogen atom concurrently is also excluded.

As an aspartyl dipeptide ester derivative used for the present invention (the second invention), the derivative represented by said formula wherein all of $R_8$, $R_9$ and $R_{10}$ denote a hydrogen atom is more preferable.

The sweetness intensity of the aspartyl dipeptide ester derivative used for the present invention (the second invention) is preferably more than 4,000 times that of sucrose.

The following contents are also included in the aspartyl dipeptide ester derivatives used for the present invention (the second invention) as a preferable derivative thereof.

[1] The derivative of the formula (2) described above, wherein $R_3$ is a hydroxyl group or a methoxy group, and $R_4$ and $R_5$ are a hydrogen atom.
[2] The derivative of the formula (2) described above, wherein $R_1$ is a hydroxyl group.
[3] The derivative of the formula (2) described above, wherein $R_1$ is a hydrogen atom.
[4] The derivative of the formula (2) described above, wherein $R_2$, $R_6$ and $R_7$ are a hydrogen atom.
[5] The above any derivative of the formula described above,

wherein $R_2$ is a hydrogen atom, a hydroxyl group or a methyl group.

The derivatives described above include those in the form of salts, for example, edible salts form such as hydrochloride salts, sodium salts, potassium salts, ammonium salts, calcium salts and magnesium salts and so on.

For the particularly preferable aspartyl dipeptide ester derivative used for the present invention (the second invention), 9 derivatives shown in the following table 2 can be enumerated. (All of $R_8$, $R_9$ and $R_{10}$ denote a hydrogen atom.)

[Table 2]

| Derivative No. | $R_1$ | $R_2$ | $R_3$ | $R_4$ | $R_5$ | $R_6$ | $R_7$ |
|---|---|---|---|---|---|---|---|
| 1 | H | OH | $OCH_3$ | H | H | H | H |
| 2 | H | H | $OCH_3$ | H | H | H | H |
| 3 | H | OH | $OCH_3$ | H | H | $CH_3$ | $CH_3$ |
| 4 | H | $CH_3$ | OH | H | H | $CH_3$ | $CH_3$ |
| 5 | H | H | $OCH_3$ | H | H | $CH_3$ | $CH_3$ |
| 6 | H | H | OH | H | H | $CH_3$ | $CH_3$ |
| 7 | OH | H | $OCH_3$ | H | H | H | H |
| 8 | H | $CH_3$ | OH | H | H | H | H |
| 9 | OH | H | OH | H | H | H | H |

A sweetener, food and drink and other sweetened product and so on, which comprise such a sweetener composition, are also contained in the present invention.

(The third invention of the present invention)

The present invention (the third invention) lies in a taste modifier comprising an aspartyl dipeptide ester derivative (which may be in the salt form) represented by the following general formula (2), more preferably the following general formula (1). Said aspartyl dipeptide ester derivatives used for the present invention (the third invention) includes those in the form of salts, and only one derivative and the mixture of not less than two derivatives are included therein.

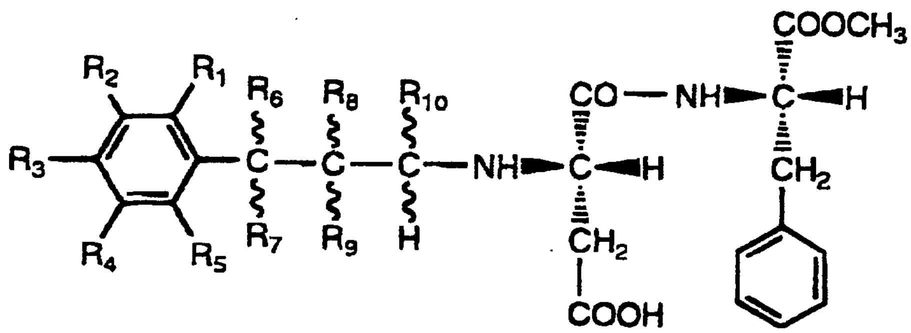

(2)

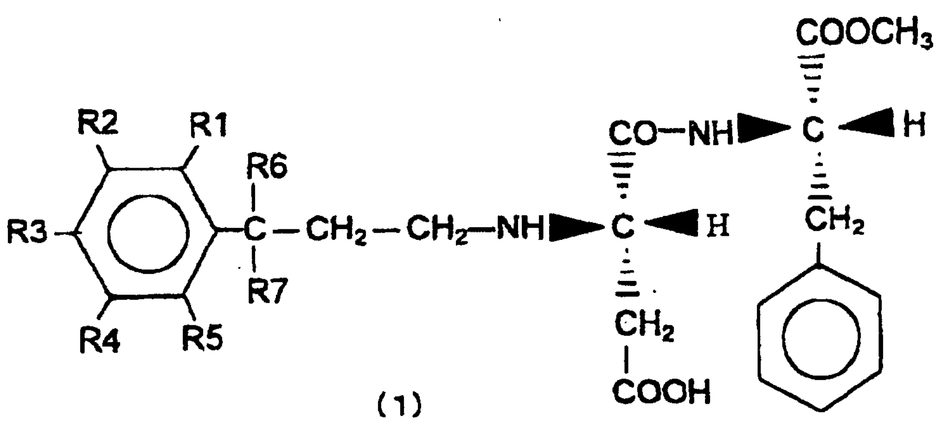

(1)

wherein in the above formulae, $R_1$, $R_2$, $R_3$, $R_4$ and $R_5$ are independent from each other, and each denotes any

one selected from the group consisting of a hydrogen atom, a hydroxyl group, an alkoxy group having 1 to 3 carbon atoms (methoxy, ethoxy, n-propoxy and so on), an alkyl group having 1 to 3 carbon atoms (methyl, ethyl, n-propyl and so on) and a hydroxyalkyloxy group having 2 or 3 carbon atoms ($O(CH_2)_2OH$, $OCH_2CH(OH)CH_3$ and so on), and herein as for $R_1$ and $R_2$, or $R_2$ and $R_3$, the respective two symbols ($R_1$ and $R_2$, or $R_2$ and $R_3$), combined together with each other, may form a methylene dioxy group ($OCH_2O$).

$R_6$, $R_7$, $R_8$, $R_9$ and $R_{10}$ are independent from each other, and each denotes a hydrogen atom or an alkyl group having 1 to 3 carbon atoms (methyl, ethyl, isopropyl and so on), respectively, herein any two substituents optionally selected from the group consisting of $R_6$, $R_7$, $R_8$, $R_9$ and $R_{10}$, combined together with each other, may form an alkylene group having 1 to 5 carbon atoms ($CH_2$, $CH_2CH_2$, $CH_2CH_2CH_2$ and so on).

When $R_6$ and $R_7$, or $R_8$ and $R_9$ denote different substituents with each other, or $R_{10}$ denotes an substituent except for a hydrogen atom, the configuration of the carbon atom to which these substituents ($R_6$ and $R_7$, $R_8$ and $R_9$ or $R_{10}$) are linked, has no restriction, and may be any one of (R), (S) and (RS) or mixture thereof, for example. In addition, wiggly lines described as the bond of $R_6$ to $R_{10}$, and an hydrogen atom with a carbon atom in said general formula (2), mean that the direction of the bond is free (is not specified).

However, the derivative in which $R_6$ denotes a hydrogen atom or a methyl group and $R_1$, $R_2$, $R_3$, $R_4$, $R_5$, $R_7$, $R_8$, $R_9$ and $R_{10}$ denote a hydrogen atom concurrently is excluded. The derivative in which $R_2$ or $R_4$ denote a methoxy group, $R_3$ denotes a hydroxyl group, $R_{10}$ denotes a hydrogen atom or a methyl group, and $R_1$, $R_4$, $R_5$, $R_6$, $R_7$, $R_8$, and $R_9$ denote a hydrogen atom concurrently is also excluded.

As an aspartyl dipeptide ester derivative used for the present invention (the third invention), the derivative represented by said formula wherein all of $R_8$, $R_9$ and $R_{10}$ denote a hydrogen atom is more preferable.

The sweetness intensity of the aspartyl dipeptide ester derivative used for the taste modifier of the present invention (the third invention) is preferably more than 4,000 times that of sucrose.

The following contents are also included in the aspartyl dipeptide ester derivatives used for the present invention (the third invention) as a preferable derivative thereof.

[1] The derivative of the formula (2) described above, wherein $R_3$ is a hydroxyl group or a methoxy group, and $R_4$ and $R_5$ are a hydrogen atom.
[2] The derivative of the formula (2) described above, wherein $R_1$ is a hydroxyl group.
[3] The derivative of the formula (2) described above, wherein $R_1$ is a hydrogen atom.
[4] The derivative of the formula (2) described above, wherein $R_2$, $R_6$ and $R_7$ are a hydrogen atom.
[5] The above any derivative of the formula described above,

wherein $R_2$ is a hydrogen atom, a hydroxyl group or a methyl group.

The derivatives described above include those in the form of salts, for example, edible salts form such as hydrochloride salts, sodium salts, potassium salts, ammonium salts, calcium salts and magnesium salts and so on.

For the particularly preferable aspartyl dipeptide ester derivative used for the present invention (the third invention), 9 derivatives shown in the following table 3 can be enumerated. (All of $R_8$, $R_9$ and $R_{10}$ denote a hydrogen atom.)

[Table 3]

| Derivative No. | $R_1$ | $R_2$ | $R_3$ | $R_4$ | $R_5$ | $R_6$ | $R_7$ |
|---|---|---|---|---|---|---|---|
| 1 | H | OH | $OCH_3$ | H | H | H | H |
| 2 | H | H | $OCH_3$ | H | H | H | H |
| 3 | H | OH | $OCH_3$ | H | H | $CH_3$ | $CH_3$ |
| 4 | H | $CH_3$ | OH | H | H | $CH_3$ | $CH_3$ |
| 5 | H | H | $OCH_3$ | H | H | $CH_3$ | $CH_3$ |
| 6 | H | H | OH | H | H | $CH_3$ | $CH_3$ |
| 7 | OH | H | $OCH_3$ | H | H | H | H |
| 8 | H | $CH_3$ | OH | H | H | H | H |
| 9 | OH | H | OH | H | H | H | H |

A product such as food and drink and a medicine which comprises such a taste modifier and of which the bitter taste is improved (removed or suppressed) is included in the present invention.

The present invention (the third invention) is particularly applicable effectively to the product containing at least one selected from the group consisting of an amino acid, a peptide, a quinine, caffeine and a mineral having a bitter taste by itself.
With regard to the mixing ratio, it is preferable to include the aspartyl dipeptide ester derivative (one kind or more) to a total amount of the product (food and drink and a medicine and so on) containing the aspartyl dipeptide ester derivative used in the present invention (the third invention), in the range of 0.2 ppm to 10000 ppm by weight.
It is usable in the form of liquid in stable, and therefore, it can exhibit or maintain the effect for the product in the form of liquid.

Preferred Embodiments

Hereafter, the preferred embodiments of the present invention were explained.

(The first invention of the present invention)

As the 9 derivatives (each is referred to as "derivatives 1 to 9") which are used in the explanation for the present invention(the first invention) are preferable in view of a high intense sweetness, the present invention (the first invention) is explained about those derivatives mainly, and however, those derivatives are representative examples thereof, and the present invention (the first invention) including those derivatives is not limited to the use of those derivatives.
The aspartyl dipeptide ester derivatives used for the present invention (the first invention) can be easily synthesized by alkylating Aspartame reductively with 3-phenylpropionaldehyde derivative, cinnamaldehyde derivative or (2-phenylethyl) alkyl ketone derivative having various substituents on the phenyl group and one or two alkyl substituents on the main chain, and a reducing agent (e.g., hydrogen/palladium carbon catalyst). Alternatively, they can be obtained by the process comprising alkylating the Aspartame derivative having a protecting group for the carboxyl group at the β position (for example, β-o-benzyl-α-L-aspartyl-L-amino acid methyl ester), which can be obtained by ordinary peptide synthesis method (Izumiya et al., Fundamentals and experiments of peptide synthesis: Maruzen, published on 1985.1.20), reductively with the 3-phenylpropionaldehyde derivative, cinnamaldehyde derivative or (2-phenylethyl) alkyl ketone derivative described above, and a reducing agent (e.g., $NaB(OAc)_3H$) (A.F.Abdel-Magid et al., Tetrahedron Letters, 31, 5595 (1990)), and then removing the protecting group, or by saturating the unsaturated bond with a reducing agent, if needed. Instead of said 3-phenylpropionaldehyde derivative, cinnamaldehyde derivative or (2-phenylethyl) alkyl ketone derivative, an acetal or ketal derivative thereof or so on can be certainly used as an aldehyde or ketone component for the reductive alkylation.
These derivatives can be easily produced by known peptide synthesis method as shown above, or according to the production examples on the derivatives 1 to 9 as shown in the Examples described later.
An another sweetener with a high intense sweetness used in the present invention (the first invention) is the sweetener with a high intense sweetness other than the compounds represented by the above general formula (2), particularly, general formula (1) (each of $R_8$, $R_9$ and $R_{10}$ in said general formula (2) denotes a hydrogen atom particularly), having a high intense sweetness in the magnification of the sweetness at least not less than approximately 10 times. As examples of such sweetener with a high intense sweetness, there are enumerated preferably Aspartame, Acesulfame K, Saccharine (including its salt form such as sodium salt), sodium cyclamate, sucralose, disodium glycyrrhizinate, Alitame, Glycyrrhizin, Stevioside (including its derivative) and Thaumatin. It is more preferable to mix and use Aspartame for improving much more the sweetness quality of the aspartyl dipeptide ester derivative used in the present invention (the first invention) among the above compounds.
There is no particular restriction to the mixed composition of the aspartyl dipeptide ester derivative and the another sweetener with a high intense sweetness used in the present invention (the first invention).
In the composition of the present invention (the first invention) at least said aspartyl dipeptide ester derivative and the another sweetener with a high intense sweetness together may be used at the same time , in any form of the use. For example, they can be used together in the form of the mixture of solid-solid, solid-liquid, liquid-liquid or so on. Further, when they are mixed during manufacture thereof, at least one or a part of both may be mixed in the form of solution, and then dried to be in the form of solid.
The effect of the improvement of the sweetness quality is influenced by the kinds of the used sweetener components and the mixed composition (ratio) and so on, and is varied depending on the used concentration thereof and the existence of the components other than the sweetener components. Therefore, as for the composition thereto, the preferable mixed composition (ratio) according to the used sweetener components and so on. It is desirable to select the most suitable composition in each system. As for the preferable concentration of the sweetness and the range of the composition, they are different according to the kinds of the used aspartyl dipeptide ester derivative and the another sweetener with a high intense sweetness, the appropriate mixed ratio in each case may be selected according to the used aspartyl dipeptide ester derivative and the another sweetener with a high intense sweetness, and further the kind and the sweetness quality of the third sweetener component, when it is used. For selecting the appropriate mixed composition (ratio), it is easy to find the most suitable concentration and composition ratio of the, components by the previous study or the preliminary experiment therefor.

When the taste quality of the sweetness is compared, it is necessary to compare it under the fixed sweetness intensity. Usually, "the sweetness ratio" or "the ratio of sweetness intensity" is adopted for an index thereof. These terms mean the ratio or the proportion dividing the sweetness intensity, when the plural of the sweetener components are included therein. The ratio of intensity or the proportion can be calculated by means of the weight ratio of sucrose obtained by calculating the weight of sucrose corresponding to the sweetness intensity of each component.

For example, when preparing the solution having a intensity of sweetness equivalent to that of 10% sucrose, wherein 80% of the 10% of intensity of sweetness is depend on the sweetener component A, for example, and the residual 20% is supplemented by the sweetener component B, the sweetness ratio is A:B=8:2. In this case, as a magnification of a sweetener component (A and B) relative to sucrose (a magnification of sweetness) is varied depending on a sweetener composition, and a concentration of the sweetener component is varied even if it is a same sweetener component (in this case, an exponential curve can be prepared and used for calculation), it is necessary to calculate the objective weight in every sweetener component and every intense sweetness. For example, if the equation for converting sweetness intensity (the exponential curve) of the sweetness component A is $Y=aX^b$, and the equation for converting sweetness intensity (the exponential curve)of the sweetness component B is $Y=cX^d$, the weight ratio of the sweetness components A and B in the mixture of the sweetness ratio A:B=8:2, can be determined by the following calculation, wherein Y denotes a concentration equivalent to that of sucrose (PSE%), and X denotes a concentration of the sweetener component (g/100ml), respectively.

Weight% of the sweetener component A

=100 x

[INV((ln(8/a))/b)]/[INV((ln(8/a))/b)+INV((ln(2/c))/d)]

(INV:inverse function of logarithm, ln:natural logarithm)

Here, if the sweetener component B is a sweetener with a low intense sweetness and so on, and the magnification of the sweetness is low: h times constant (there is no sweetness intensity curve), the weight% may be determined as follows.

Weight% of the sweetener component A

=100 x [INV((ln(8/a))/b)]/[INV((ln(8/a))/b)+2/h]

Thus, a skilled person in the art can calculate it easily in another system as described above.

When Aspartame is used, the composition of the sweetness quality closer to that of sucrose can be produced by including it in the mixed composition with the aspartyl dipeptide ester derivative, at the ratio of preferably at least not less than 5%, more preferably approximately 5 to 90%, further more preferably approximately 20 to 90% therein by the ratio of sweetness intensity. As the sweetness intensity varies depending on the kind of said derivative, the suitable ranges of the weigh ratio can be determined respectively in each case. However, if the weight ratio applicable to all cases regardless of the kind of the derivative and a mixing state is represented, Aspartame can be mixed thereto, in the range of approximately 5 to 99.9%, more preferably approximately 10 to 99.9%, and further more preferably approximately 20 to 99.8% by weight to a total amount of one or more said derivatives and Aspartame. Particularly, in case of derivative 1 alone, Aspartame can be mixed thereto in the range of preferably approximately 60 to 99.8%, more preferably approximately 94 to 99.8% by weight.

In case of derivative 2, Aspartame can be mixed thereto in the range of preferably approximately 25 to 99.7%, more preferably approximately 77 to 99.7% by weight.

Thus, the preferable ranges of Aspartame in the composition with other derivatives used in the present invention (the first invention), also can be determined.

On the other hand, with regard to also said another sweetener with a high intense sweetness other than Aspartame, the suitable range of composition can be determined according to the kind thereof.

Further, when another sweetener with a high intense sweetness other than Aspartame is used, the preferable

mixed composition (ratio) can be selected by studying the ratio of sweetness intensity as described above, and it can be mixed in the range of 1 to 99.9% by weight according to the components to be mixed.

As is described previously, when the sweetness quality is compared, it is necessary to fix the sweetness intensity. It is generally preferable to use the sweetness intensity equivalent to that of 10% sucrose (which is referred to as "PSE 10%". PSE: abbreviation of Point of Subjective Equality) in case of a drink such as a cola drink, and the sweetness intensity equivalent to that of 5% sucrose (PSE 5%) in case of black tea, coffee and so on. In the present invention (the first invention), the comparative evaluation was performed by using the solution equivalent to 10% sucrose.

The sweetener composition of the present invention (the first invention) can be used as a sweetener. In this case, the composition may contain at least one of said derivatives and at least one of the another sweeteners with a high intense sweetness. As is described previously, both components may be in any form of both mixing. The form of mixing is not limited. The mixing form such as liquid-liquid . and solid-solid (powder mixing) and other various mixing forms are adopted.

Further, a different sweetener component (the third sweetener component such as sugar, sugar alcohol and so on), and the necessary component other than a sweetener component, . for example, salt such as sodium chloride or so on can be mixed. Incidentally, sodium chloride can improve a sweetness quality of said aspartyl dipeptide ester derivative by using therewith.

When the sweetener composition of the present invention (the first invention) is used as a sweetener, a necessary carrier, a bulking agent and/or a filler for a sweetener can be used. In this case, a carrier, a bulking agent, a filler and so on for a sweetener which are known or used previously, for example.

As such a carrier, a general sugar (sucrose, invert sugar, isomerized sugar, glucose, fructose, lactose, malt sugar, D-xylose and isomerized lactose and so on), sugar alcohol (maltitol [reduced maltose syrup and so on], sorbitol, mannitol, erythritol, xylitol, lactitol [reduced lactose and so on], palatinit, and hydrogenated starch hydrolysate [reduced starch syrup and so on] and so on), oligosaccharide (fructooligosaccharide [neosugar and so on], ma tooligosaccharide [linear chain oligosaccharide and so on], isomaltooligosaccharide [branched chain oligosaccharide and so on], galactooligosaccharide, soy been oligosaccharide, lactooligosaccharide and so on), a derivative of sucrose (sucrose binding starch sugar and so on [coupling sugar: glucosylsucrose and so on] and so on), palatinose [isomaltulose and so on], trehalose and so on), polysaccharide (glucomannan and so on.

), dietary fiber (enzyme decomposition product of guar gum [hydrolysate of galactomannan and so on], non-digestible dextrin [dietary fiber containing dextrin and so on], polydextrose and so on), and starch (dextrin, soluble starch, modified starch and so on) can be used. When such carriers are used, a single compound included in these compounds or a mixture of plural compounds therein can be suitably selected and used.

The sweetener composition of the present invention (the first invention), further can be used as a sweetener for various products, that is the product such as food and drink which is in need of sweet taste, for example, a confectionary (an ice cream or a sherbet, a jelly, a cake, a candy), bread, chewing gum, a sanitary product, cosmetics (including an oral composition such a tooth paste), a chemical (medicine) and an animal product other than human. The sweetener composition of the present invention (the first invention) can be used both in the form of such sweetened products, and also in a method for imparting the sweetness to the product which is in need of sweetness, and also they are contained naturally in the present invention. In this case, objective sweetness can be easily imparted by adding or including said sweetener composition to or in the product such as food and drink and so on which is in need of sweetness or the intermediate product during its manufacture. With regard to the method for using the sweetener composition (for example, the method of adding or including it), any method which is known as a method used for using a sweetener component for a sweetener or a method for imparting the sweetness (sweet taste) and so on can be used.

According to the use of the sweetener composition of the present invention (the first invention), a natural and well-balanced sweetness quality which is closer to that of sucrose, can be obtained.

(The second invention of the present invention)

With regard to the aspartyl dipeptide ester derivatives represented by said general formula (2), particularly (1), which are used for the present invention (the second invention), especially aforementioned 9 derivatives (referred to as "derivative 1" to "derivative 9" respectively) are preferable in view of a high intense sweetness. The present invention (the second invention), therefore, is explained mainly about these derivatives, and however, the present invention (the second invention) is not limited to the use of these derivatives.

The aspartyl dipeptide ester derivatives used for the present invention (the second invention) can be easily synthesized by alkylating Aspartame reductively with 3-phenylpropionaldehyde derivative, cinnamaldehyde derivative or (2-phenylethyl) alkyl ketone derivative having various substituents on the phenyl group and one or two alkyl substituents on the main chain, and a reducing agent (e.g., hydrogen/palladium carbon catalyst). Alternatively, they can be obtained by the process comprising alkylating the Aspartame derivative having a protecting group for the carboxyl

group at the β position (for example, β-o-benzyl-α-L-aspartyl-L-amino acid methyl ester), which can be obtained by ordinary peptide synthesis method (Izumiya et al., Fundamentals and experiments of peptide synthesis: Maruzen, published on 1985.1.20), reductively with the 3-phenylpropionaldehyde derivative, cinnamaldehyde derivative or (2-phenylethyl) alkyl ketone derivative described above, and a reducing agent (e.g., $NaB(OAc)_3H$) (A.F.Abdel-Magid et al., Tetrahedron Letters, 31, 5595 (1990)), and then removing the protecting group, or by saturating the unsaturated bond with a reducing agent, if needed. Instead of said 3-phenylpropionaldehyde derivative, cinnamaldehyde derivative or (2-phenylethyl) alkyl ketone derivative, an acetal or ketal derivative thereof or so on can be certainly used as an aldehyde or ketone component for the reductive alkylation.

These derivatives can be easily produced by known peptide synthesis method as shown above, or according to the production examples on the derivatives 1 to 9 as shown in the Examples described later.

Sugar, sugar alcohol and oligosaccharide used in the present invention (the second invention) (hereafter, it may be referred to as "sugar and so on used in the present invention" or "sugar and so on used in the present invention (the second invention)) are explained as follows.

With regard to sugar, among the sugars any sugar which has a sweet taste and is soluble in water, is preferably used. For example, it comprises sucrose (including derivative thereof), invert sugar, isomerized sugar, glucose, fructose, lactose, malt sugar, D-xylose and isomerized lactose. The derivative of sucrose comprises, for example, sucrose binding starch sugar (including coupling sugar, glucosylsucrose and so on), palatinose (including isomaltulose and so on) and trehalose and so on.

The term "sugar alcohol" means the reduced sugar, and the term "oligosaccaride" means the polysaccharide which has several basic frame of monosaccharide such as glucose and fructose. With regard to sugar alcohol, there are enumerated maltitol, sorbitol, mannitol, erythritol, xylitol, lactitol, palatinit, and reduced starch sugar. With regard to oligosaccharide, there are enumerated fructooligosaccharide, maltooligosaccharide, isomaltooligosaccharide, galactooligosaccharide, soy been oligosaccharide and lactooligosaccharide.

Either one or plural of these compounds can be used for the present invention (the second invention).

Among the above described compounds, in view of improving the sweetness quality of the aspartyl dipeptide ester derivative(s) used in the present invention (the second invention), it is preferable to mix and use sucrose. And in view of the superiority of the same effect, sugar alcohol such as erythritol, maltitol, sorbitol, xylitol and so on, is preferably used.

In the present invention (the second invention), there is no particular restriction to the mixed composition (ratio) of the aspartyl dipeptide ester derivative(s) and sugar and so on used in the present invention (the second invention).

In the composition of the present invention (the second invention), at least said aspartyl dipeptide ester derivative and any one of the compounds included in the group consisting of said sugar, sugar alcohol and oligosaccharide may be used together, in any form of the combination. For example, they can be used together , for example, in the form of the mixture of solid-solid (powder and so on), solid-liquid and liquid-liquid. Further, when they are mixed during manufacture, at least one or a part of both may be mixed in the form of solution, and then dried to be in the form of solid.

The effect of the improvement of the sweetness quality is influenced by the kinds of the used sweetness components and the mixed composition (ratio) and so on, and is varied depending on the used concentration thereof and the existence of the components other than the sweetness components. Therefore, as for the preferable mixed composition (ratio) it is necessary to adjust appropriately according to the used sweetness components and so on. It is desirable to select the most suitable composition in each system. As for the preferable concentration of the sweetness and the range of the composition, they are different according to the kinds of the used aspartyl dipeptide ester derivative and the sugar and so on used in the present invention (the second invention), the appropriate mixed ratio may be selected in each case according to the used aspartyl dipeptide ester derivative and the sugar and so on used in the present invention (the second invention), and further the kind and the sweetness quality of the third sweetness component, when it is used. For selecting the appropriate mixed composition (ratio), it is easy to find the most suitable concentration and composition ratio of components by the previous study or the preliminary experiment therefor.

When the taste quality of the sweetness is compared, it is necessary to compare it under the fixed sweetness ratio. Usually, "the sweetness ratio" or "the sweetness proportion" is adopted for an index thereof. These terms mean the ratio or the proportion dividing the intensity of sweetness, when the plural of the sweetener components are included therein. The ratio of intensity or the proportion can be calculated by means of the weight ratio of sucrose obtained by calculating the weight of sucrose corresponding to the intensity of sweetness of each component.

For example, when preparing the solution having a intensity of sweetness equivalent to that of 10% sucrose, wherein 80% of the 10% of intense sweetness is depend on the sweetener component A, for example, and the residual 20% is supplemented by the sweetener component B, the sweetness ratio is A:B=8:2. In this case, as a magnification of a sweetener component (A and B) relative to sucrose (a magnification of sweetness) is varied depending on a sweetener composition, and a concentration of the sweetener component is varied even if it is a same sweetener component (in this case, an exponential curve can be prepared and used for calculation), it is necessary to calculate

the objective weight in every sweetener component and every intensity of sweetness. For example, if the equation for converting sweetness intensity (the exponential curve) of the sweetness component A is $Y=aX^b$, and the equation for converting sweetness intensity (the exponential curve)of the sweetness component B is $Y=cX^d$, the weight ratio of the sweetness components A and B in the mixture of the sweetness ratio A:B=8:2, can be determined by the following calculation, wherein Y denotes a concentration equivalent to that of sucrose (PSE%), and X denotes a concentration of the sweetener component (g/100ml), respectively.

Weight% of the sweetener component A

=100 x [INV((ln(8/a))/b)]/[INV((ln(8/a))/b)+INV((ln(2/c))/d)]

(INV:inverse function of logarithm, ln:natural logarithm)

Here, if the sweetener component B is a sweetener with a low intensity of sweetness and so on, and the magnification of the sweetness is low: h times constant (there is no sweetness intensity curve), the weight% may be determined as follows.

Weight% of the sweetener component A

=100 x [INV((ln(8/a))/b)]/[INV((ln(8/a))/b)+2/h]

Thus, a skilled person in the art can calculate it easily in another system as described above.

When sucrose is used in the present invention (the second invention), concerning the ratio used of sucrose, the composition having the sweetness quality closer to that of sucrose can be produced by including it in the mixed composition with the aspartyl dipeptide ester derivative, in the ratio of preferably at least not less than 5%, more preferably approximately 5 to 95%, further more preferably approximately 20 to 90% in the mixture by the ratio of sweetness intensity (sweetness ratio). As the sweetness intensity of the derivative(s) used in the present invention (the second invention) is large, when the ratio used of the derivatives is represented by weight ratio, said derivatives exist in said mixture in the order of ppm (parts per million) . For example, when they are used by mixing with sucrose, said derivatives may be mixed, in the range of preferably approximately 0.5 ppm to 5000 ppm (by weight), and more preferably approximately 1 ppm to 1000 ppm (by weight) to a total amount of said derivative and sucrose.

Particularly, when derivative 1 is used by mixing with sucrose, derivative 1 may be mixed thereto, in the range of preferably approximately 5 ppm to 850 ppm (by weight), and more preferably approximately 5 ppm to 200 ppm (by weight) to a total amount of derivative 1 and sucrose. And, when derivative 2 is used by mixing with, for example, sucrose, derivative 2 may be mixed thereto, in the range of preferably approximately 6 ppm to 4000 ppm (by weight), and more preferably approximately 6 ppm to 1000ppm to a total amount of derivative 2 and sucrose.

In addition, when sugar alcohol, particularly at least one of erythritol, maltitol, sorbitol, and xylitol is used in the present invention (the second invention), concerning the ratio used thereof, the composition having the sweetness quality closer to that of sucrose can be produced by including it in the mixed composition with the aspartyl dipeptide ester derivative, in the ratio of preferably at least not less than 5%, more preferably approximately 5 to 95%, further more preferably approximately 20 to 90% therein by the ratio of sweetness intensity (sweetness ratio). When the aspartyl dipeptide ester derivative is used by mixing with sugar alcohol, and when represented by weight ratio, said derivative may be mixed thereto, in the range of preferably approximately 0.5 ppm to 5000 ppm (by weight), and more preferably approximately 1 ppm to 1000 ppm (by weight) to a total amount.

Particularly, when derivative 1 is used by mixing with sugar alcohol, derivative 1 may be mixed, in the range of preferably approximately 1 ppm to 3000 ppm (by weight), and more preferably approximately 1 ppm to 100 ppm (by weight) to a total amount thereof. And, when derivative 2 is used by mixing with sugar alcohol as the same way, derivative 2 can be mixed, in the range of preferably approximately 1 ppm to 1500 ppm (by weight), and more preferably approximately 1 ppm to 300 ppm (by weight) thereto.

To a total amount of the aspartyl dipeptide ester derivative and sugar, sugar alcohol and oligosaccharide present, it is preferably to contain said derivative in the range of 0.5 ppm to 5000 ppm (by weight).

When the aspartyl dipeptide ester derivative used in the present invention (the second invention) are used by mixing with at least one of the compounds contained in the group consisting of sugar, sugar alcohol and oligosaccharide other than those described above, the high-quality of sweetness closer to that of sucrose can be imparted by containing the present compound(s) contained in the group consisting of sugar, sugar alcohol and oligosaccharide in the same manner, in the ratio of preferably not less than 5%, more preferably approximately 5 to 95%, and further

more preferably approximately 20 to 90% by sweetness intensity to a total amount thereof.

Further, when the sweetness quality is compared, it is necessary to fix the sweetness intensity. It is generally preferable to use the sweetness intensity equivalent to that of 10% sucrose (which is referred to as "PSE 10%". PSE: abbreviation of Point of Subjective Equality) in case of a drink such as a cola drink, and the sweetness intensity equivalent to that of 5% sucrose (PSE 5%) in case of black tea, coffee and so on. In the present invention (the second invention), the comparative evaluation was performed by using the solution equivalent to 10% sucrose.

The sweetener composition of the present invention (the second invention) can be used as a sweetener. In this case, the composition may contain at least one of said derivatives and at least one of such other sweeteners.

Further, different sweetener component(s) (the third sweetener component and fourth sweetener component, and so on: other sweetener with a high intense sweetness such as Aspartame), and the necessary component other than a sweetener component, for example, salt such as sodium chloride and so on can be mixed.

When the sweetener composition of the present invention (the second invention) is used as a sweetener, a necessary carrier, a filler and/or an excipient for a sweetener can be used. In this case, a carrier, a filler, an excipient and so on for a sweetener which are known or used previously, for example.

As such a carrier, a general sugar (sucrose, invert sugar, isomerized sugar, glucose, fructose, lactose, malt sugar, D-xylose and isomerized lactose and so on), sugar alcohol (maltitol [reduced maltose syrup and so on], sorbitol, mannitol, erythritol, xylitol, lactitol [reduced lactose and so on], palatinit, and hydrogenated starch hydrolysate [reduced starch syrup and so on] and so on), oligosaccharide (fructooligosaccharide [neosugar and so on], maltooligosaccharide [linear chain oligosaccharide and so on], isomaltooligosaccharide [branched chain oligosaccharide and so on], galactooligosaccharide, soy been oligosaccharide, lactooligosaccharide and so on), a derivative of sucrose (sucrose binding starch sugar and so on [coupling sugar: glucosylsucrose and so on]and so on), palatinose [isomaltulose and so on], trehalose and so on), polysaccharide (glucomannan and so on), dietary fiber (enzyme decomposition product of guar gum [hydrolysate of galactomannan and so on], non-digestible dextrin [dietary fiber containing dextrin and so on], polydextrose and so on), and starch (dextrin, soluble starch, modified starch and so on) can be used. When such carriers are used, a single compound included in these compounds or a mixture of plural compounds therein can be suitably selected and used.

When said sugar and so on are used as a carrier, the effect of the sugar and so on used for improving the sweetness quality in the present invention (the second invention) is obtained, and thus, the sugar and so on used in this way, have both effects (Carrier and Improvement of sweetness quality).

The sweetener composition of the present invention (the second invention), further can be used as a sweetener for various products, that is the product such as food and drink which is in need of sweet taste, for example, a confectionary (an ice cream or a sherbet, a jelly, a cake, a candy), bread, chewing gum, a sanitary product, cosmetics (including an oral composition such a tooth paste), a chemical (medicine) and an animal product other than human. The sweetener composition of the present invention (the second invention) can be used both in the form of such sweetened products, and also in a method for imparting the sweetness to the product which is in need of sweetness, and also they are contained naturally in the present invention. In this case, objective sweetness can be easily imparted by adding or including said sweetener composition to or in the product such as food and drink and so on which is in need of sweetness or the intermediate product during its manufacture. With regard to the method for using the sweetener composition (for example, the method of adding or including it), any method which is known as a method used for using a sweetener component for a sweetener or a method for imparting the sweetness (sweet taste) and so on can be used.

According to the use of the sweetener composition of the present invention (the second invention), a natural and well-balanced sweetness quality which is closer to that of sucrose, can be obtained.

(The third invention of the present invention)

With regard to the aspartyl dipeptide ester derivatives represented by said general formula (2), particularly (1), which are used for the present invention (the third invention), especially aforementioned 9 derivatives (referred to as "derivative 1" to "derivative 9" respectively) are preferable in view of a high effect of correcting the taste. The present invention (the third invention), therefore, is explained mainly about these derivatives, and however, the present invention (the third invention) is not limited to the use of these derivatives.

The aspartyl dipeptide ester derivatives used for the present invention (the third invention) can be easily synthesized by alkylating Aspartame reductively with 3-phenylpropionaldehyde derivative, cinnamaldehyde derivative or (2-phenylethyl) alkyl ketone derivative having various substituents on the phenyl group and one or two alkyl substituents on the main chain, and a reducing agent (e.g., hydrogen/palladium carbon catalyst). Alternatively, they can be obtained by the process comprising alkylating the Aspartame derivative having a protecting group for the carboxyl group at the β position (for example, β-o-benzyl-α-L-aspartyl-L-amino acid methyl ester), which can be obtained by ordinary peptide synthesis method (Izumiya et al., Fundamentals and experiments of peptide synthesis: Maruzen,

published on 1985.1.20), reductively with the 3-phenylpropionaldehyde derivative, cinnamaldehyde derivative or (2-phenylethyl) alkyl ketone derivative described above, and a reducing agent (e.g., $NaB(OAc)_3H$) (A.F.Abdel-Magid et al., Tetrahedron Letters, 31, 5595 (1990)), and then removing the protecting group, or by saturating the unsaturated bond with a reducing agent, if needed. Instead of said 3-phenylpropionaldehyde derivative, cinnamaldehyde derivative or (2-phenylethyl) alkyl ketone derivative, an acetal or ketal derivative thereof or so on can be certainly used as an aldehyde or ketone component for the reductive alkylation.

These derivatives can be easily produced by known peptide synthesis method as shown above, or according to the production examples on the derivatives 1 to 9 as shown in the Examples described later.

There is no restriction to the kind and the mixed amount of substances of bitter taste which can be improved by the taste modifier of the present invention (the third invention) . Any form of the combination of the taste modifier and the substance of bitter taste may be used. They may be used together in the form of the mixture of solid-solid (powder and so on), and liquid-liquid. When they are mixed during manufacture, at least one of both which is in the solutions, may be mixed together homogeneously, and then dried to be in the form of solid.

As an example of a bitter taste which can be improved by the taste modifier of the present invention (the third invention), arginine, valine, leucine, isoleucine, methionine, histidine, ornithine, proline, lysine, another amino acid having a bitter taste, a peptide having a bitter taste, quinine, caffeine, calcium ion, another mineral having a bitter taste, and a bitter taste containing substance such as various herbal medicines and so on can be enumerated. Particularly, as an effective ingredient having a bitter taste, there are enumerated for example, vinpocetine, fursultiamine, and fursultiamine hydrochloride, sefucaneldaroxicete, cefotiam hexetil hydrochloride, lenampicillin hydrochloride, bacampicillin hydrochloride, talampicillin hydrochloride, pivmecillinam hydrochloride, oxeladin tannate, clobutinol hydrochloride, berberine hydrochloride, propantheline bromide, papaverine hydrochloride, ticlopidine hydrochloride, chlorpromazine hydrochloride, and sultamicillin tosylate as described in for example Japanese Patent Kokai Publication JP-A-H4-327529, anhydrous caffeine, diprophylline, diphenhydramine salicylate, chlorpheniramine maleate, pyridoxine hydrochloride, dimenhydrinate, meclizine hydrochloride, methylephedrine hydrochloride, guaiacol potassium sulfonate, guanethidine, chlorhexidine hydrochloride, dihydrocodeine phosphate, ephedrine hydrochloride, spironolactone tegafur, erythromycin stearate, alacepril, sodium valproate, meclofenoxate hydrochloride, chloramphenicol, aminophylline, erythromycin, calcium hopantate, calcium pantothenate, phenobarbital, cimetidine, etilefrine hydrochloride, pirenzepine hydrochloride, butyl scopolamine hydrochloride, dilteazem hydrochloride, enoxacin, piromidate trihydrate, propranolol hydrochloride, flufenamic acid, chlorpromazine, digitonin, promethazine hydrochloride, metoclopramide hydrochloride, ofloxacin, sulpyrine, acetaminophen, aspirin, ibuprofen, benzydamine hydrochloride, alprenolol hydrochloride, bifemelane hydrochloride, lidocaine, diphenhydramine hydrochloride, sodium tolmetin, nortriptyline hydrochloride, and loperamide hydrochloride as described in for example Jananese Patent Kokai Publication JP-A-4-327526, and, azelastine hydrochloride, bifemelane hydrochloride, quinidine sulfate, and s-(+)-(2-chlorophenyl)-3-cyclopropanecarbonyl-8,11-dimethyl-2,3,4,5-tetrahydro-8H-pyrido-[4,3;4,5]thieno[3,2-f][1,2,4]triazoro[4,3-a][1,4]diazepin as described in for example Japanese Patent Kokai Publication JP-A-4-282312, and further (+)-(5R,6S)-6-[(R)-1-hydroxyethyl]-3-(3-pyridyl)-7-oxo-4-thia-1-azabicyclo[3,2,0]hepto-2-ene-2-carboxylic acid acetoxymethyl ester, and so on as described in Japanese Patent Kokai Publication JP-A-4-257457 (refer to JP Patent Kokai Publication JP-A-6-298668).

When the bitter taste is included in the product such as food and drink and a medicine and so on, the effect of the present invention (the third invention) is obtained by using either one or plural of these compounds as described above. Depending on the content of the substance of bitter taste, almost all or a part of the bitter taste can be removed, or the bitter taste can be suppressed (decreased). If the content of the substance of bitter taste is too high, the complete removal of bitter taste may be impossible even by adding some amount of the derivative used in the present invention (the third invention). In this case, the effect of removing a part of bitter taste or suppressing (decreasing) the bitter taste is expressed by mixing an appropriate amount of the derivative. Thus, the use of said derivative for the effect of removing a part of bitter taste or suppressing (decreasing) the bitter taste is actually included in the present invention.

When the aspartyl dipeptide ester derivative used for the present invention (the third invention), is used as a taste modifier in the product such as food and drink, a medicine and so on, one or plural of the derivatives can be used therefor. The ratio of the aspartyl dipeptide ester derivative (one kind or more) included in the finished product to the total amount of the product, varies, also depending on the kind of the product. It is preferably in the range of approximately 0.2 weight ppm to 10000 weight ppm, and more preferably in the range of approximately 1 weight ppm to 5000 weight ppm thereto. Particularly, according to the magnification of the sweetness of the derivative used, the appropriate content thereof must be selected. When the concentration of the derivative used is too low, the effect of correcting the bitter taste is not sufficient, and when it is too high, the taste of the product, particularly when it is used for drink, is problematic (in taste) by the excess intensity of the sweetness.

For example, when the solution of PSE 10% (PSE:Point of Subjective Equality) is prepared by using derivative A having 50000 times of magnification of sweetness intensity, 10/50000 g (vs. 100g solution) of derivative A may be used ( the content of derivative A in the solution is equivalent to 2 ppm, and that in the form of solid is 200 ppm). On the other hand, when the solution of PSE 5% is prepared by using derivative B having 4000 times of magnification of

sweetness intensity, 5/4000 g (vs. 100g solution) of derivative B may be used ( the content of derivative B in the solution is equivalent to 12.5 ppm, and that in the form of solid is 1250 ppm).

The effect of the taste modifier of the present invention (the third invention) is observed extremely, particularly in the form of liquid without any particular restriction. Further, any form of the taste modifier such as powder, granule, tablet, another solid state, paste and so on can be used.

When the aspartyl dipeptide ester derivative used in the present invention (the third invention) is used as a taste modifier by adding it to the product such as food and drink and a medicine during manufacture, there is no particular restriction about the time and method of addition thereof.

The method for correcting a taste by adding or including said taste modifier to or in such product such as food and drink and a medicine and so on or the intermediate thereof during manufacture, is properly included in the . present invention. With regard to the method for using these taste modifiers (for example, the method of adding or including them), any method which is known so far as a method used when using a taste modifier or an effective component thereof and so on can be applied thereto and used therefor.

## Examples

Hereinafter, the present invention is explained in more detail by reference to Examples, Comparable Examples and further Production Examples of the aspartyl dipeptide ester derivatives which are used for the present invention.

(First invention of the present invention)

(Example 1) Measurement of the magnification of sweetness intensity

An aqueous solution was prepared by diluting derivative 2 to be PSE 10% concentration (15.5 mg/1000 ml = 10/6500 g/100 ml), assuming that the intensity of sweetness of derivative 2 was 6500 times that of sucrose. Separately, aqueous sucrose solutions having sucrose concentrations of (a)6.94%, (b)8.33%, (c)10%, (d)12%, and (e)14.4% were prepared. The sensory evaluation was performed by determining which sucrose solution was closest to the solution of derivative 2 in the sweetness intensity. The result of calculation of the average of points of 20 panelists was 2.25 point.

The intensity of sweetness of the solution of derivative 2 was 8.75% according to the following equation: (10.0-8.33) x 0.25 +8.33=8.75. Accordingly, the intense sweetness of derivative 2 was 5600 (=8.75/0.00155) times that of sucrose. According to the same experiment, the intensity of sweetness of derivative 1 was 22600 times that of sucrose. Furthermore, the magnification of sweetness intensity of other derivatives (3 to 9) can be determined by the same method.

And the magnification of sweetness intensity in the cola drink can be also determined by the same method compared to the control solution of cola drink containing 10% sucrose.

The composition of cola drink is as follows.

| | |
|---|---|
| Citric acid (crystalline) | 0.25g/1000ml |
| Sodium citrate | 0.10g/1000ml |
| 85% Phosphoric acid | 0.3g/1000ml |
| Cola base | 2ml/1000ml |
| Cola essence | 1ml/1000ml |
| Sweetener (sample) | Prescribed amount |

As for the concentration of the references, the sucrose concentrations of previous (a) to (e) were used. In consequence, the magnification of sweetness intensity in the cola drink of derivative 1 was 22600 times, and that of derivative 2 was 4900 times.

(Example 2) The taste characteristics (In aqueous solution)

The following experiments were performed using the water obtained by ion exchanging and further distilling water.

An aqueous solution of each of derivatives 1 to 9 having a sweetness level equivalent to that of PSE 10% was prepared, and compared with aqueous 10% sucrose solution with respect to the following 9 items, i.e., "onset of

the taste", "round taste", "clear taste", "lingering taste", "peculiar taste", "heavy taste", "bitter taste", "astringent taste" and "irritation". The results were determined as 5 levels (-2 point: very weak, -1 point: a little weak, 0 point: same, +1 point: a little strong, +2 point: very strong) by 8 panelists, and the average points were calculated. The sample solutions of the derivatives equivalent to PSE 10%, were prepared by using the magnification of sweetness intensity measured in Example 1, and the amounts of each derivatives to be used were as follows.

| Sample | Amount equivalent to PSE 10% (mg/1000ml) |
|---|---|
| Derivative 1 | 4.5 |
| Derivative 2 | 17.9 |
| Derivative 3 | 2.3 |
| Derivative 4 | 2.3 |
| Derivative 5 | 11.9 |
| Derivative 6 | 6.7 |
| Derivative 7 | 9.0 |
| Derivative 8 | 5.5 |
| Derivative 9 | 12.5 |

(Results of the taste characteristics)

All of the derivatives were extremely weak in the onset of the taste, very weak in round and clear taste, extremely strong in lingering taste, and very strong in peculiar, heavy, bitter and astringent taste, and irritation.

Next, the total of the absolute value of the deviation from sucrose in each of the evaluation items consisting of the onset of the taste, the round taste, the clear taste, the lingering taste, the peculiar taste, the heavy taste, the bitter taste, the astringent taste and the irritation was divided by 9 (total number of items) to calculate the similarity (which is referred to as "similarity index"). The smaller the similarity index becomes, the better the taste balances, and the closer the taste is to the taste characteristics (quality) of sucrose. The total taste becomes preferable. The similarity indexes of each derivatives are shown as follows.

| Sample | Similarity index |
|---|---|
| Derivative 1 | 1.17 |
| Derivative 2 | 0.63 |
| Derivative 3 | 1.04 |
| Derivative 4 | 0.93 |
| Derivative 5 | 1.38 |
| Derivative 6 | 0.93 |
| Derivative 7 | 0.86 |
| Derivative 8 | 0.77 |
| Derivative 9 | 0.77 |

(Example 3) The taste characteristics (In cola drink)

The following experiments were performed in the same way as that of Example 2. Instead of aqueous solution, the cola drink of each of derivatives 1 to 9 having a sweetness level equivalent to that of PSE 10% was prepared, and compared with the cola drink containing 10% sucrose. The evaluation method and so on were same as that of Example 2.

When carbon dioxide is blown in the cola drink, the carbonated cola drink is prepared, however, the taste of the cola drink can be compared more easily in that without carbon dioxide. Thus the sensory evaluation of non-carbonated cola drink was performed.

The composition of the cola drink employed was same as that of Example 1.

| Sample | Amount equivalent to PSE 10% (mg/1000ml) |
|---|---|
| Derivative 1 | 4.5 |
| Derivative 2 | 20.4 |
| Derivative 3 | 2.7 |
| Derivative 4 | 3.4 |
| Derivative 5 | 12.5 |
| Derivative 6 | 7.1 |
| Derivative 7 | 9.4 |
| Derivative 8 | 6.3 |
| Derivative 9 | 13.3 |

(Results of the taste characteristics)

All of the derivatives were extremely weak in the onset of the taste, very weak in round and clear taste, extremely strong in the lingering taste, and very strong in peculiar, heavy, bitter and astringent taste, and irritation.

The similarity indexes were calculated as those of aqueous solutions. The smaller the similarity index becomes, the better the taste balances, and the closer the taste is to the taste characteristics (quality) of sucrose. The total taste becomes preferable. The similarity indexes of each derivatives are shown as follows.

| Sample | Similarity index |
|---|---|
| Derivative 1 | 0.78 |
| Derivative 2 | 0.80 |
| Derivative 3 | 1.11 |
| Derivative 4 | 1.04 |
| Derivative 5 | 1.34 |
| Derivative 6 | 0.81 |
| Derivative 7 | 0.90 |
| Derivative 8 | 1.00 |
| Derivative 9 | 1.00 |

(Example 4) Improvement of taste by Aspartame

The mixture with Aspartame of each of derivatives 1 to 9 was prepared and the sensory evaluation of the taste was performed by the same method as described in Example 3.

The sweetener composition having a sweetness level equivalent to that of PSE 10% was mixed with the following sweetness ratio.

| Derivatives 1 to 9 | : | Aspartame |
|---|---|---|
| 8 | : | 2 |
| 5 | : | 5 |
| 2 | : | 8 |

Equations for calculating sweetness intensity in the cola drink (pH2.8, 20°C) are as follows, wherein Y denotes the concentration equivalent to that of sucrose (g/100ml) and X denotes the concentration of the sweetener (g/100ml),

respectively.

| Sample | Calculating equation |
|---|---|
| Derivative 1 | $Y=212300 \times X^{1.29}$ |
| Derivative 2 | $Y = 1890 \times X^{0.847}$ |
| Derivative 6 | $Y = 9520 \times X^{0.947}$ |
| Derivative 8 | $Y = 3940 \times X^{0.812}$ |

In case of derivative 1,

When Y=9.5, X=0.000425 g/100ml
When Y=8, X=0.00037 g/100ml
When Y=5, X=0.00026 g/100ml
When Y=2, X=0.00013 g/100ml
When Y=1, X=0.000074 g/100ml

With regard to Aspartame, the values at 20°C, pH2.8 were calculated, that is

When Y=0.5, X=0.00072
When Y=2, X=0.0053
When Y=5, X=0.0202
When Y=8, X=0.0398
When Y=9, X=0.04726

In all cases, as the ratio of Aspartame became larger, the similarity index became smaller compared to the taste of each of the derivatives alone (refer to Example 3) and it was confirmed to be closer to the taste of sucrose.
When the cola drink having a sweetness level equivalent to that of PSE 10% is prepared by mixing derivative 1 and Aspartame, the ratio (%) of Aspartame included in the total amount of the mixture by weight is calculated as follows.
In case of the sweetness ratio of derivative 1:Aspartame=9.5:0.5,
0.000425 (derivative 1) + 0.00072 (Aspartame) = 0.00115 g/100ml: the percentage content of Aspartame is 62.6% by weight.
In the same way, in case of the sweetness ratio of derivative 1:Aspartame=8:2,
0.00037 (derivative 1) + 0.0053 (Aspartame) = 0.00567 g/100ml: the percentage content of Aspartame is 93.5% by weight. In the same way, when the sweetness ratio of derivative 1:Aspartame=2:8, the percentage content of Aspartame is 99.7% by weight.
The same experiments as described above can be performed by a skilled person in the art for calculating those values of each of derivatives 2 to 9 instead of derivative 1, and those values of the another sweeteners with a high intense sweetness other than Aspartame.

(Example 5) Improvement of taste by another sweetener with a high intense sweetness other than Aspartame

The mixture with each of Acesulfame K, Sodium Saccharine, Alitame, disodium glycyrrhizinate, Stevioside (including its derivative) and Thaumatin of each of derivatives 1 to 9 was prepared and the sensory evaluation for the taste of the mixture was performed by the same method as described in Example 3.
The sweetener composition having a sweetness level equivalent to that of PSE 10% was prepared with the following sweetness ratio.

| Derivatives 1 to 9 | : | Another sweetener with a high intense sweetness |
|---|---|---|
| 5 | : | 5 |
| 8 | : | 2 |
| 9 | : | 1 |

With regard to Acesulfame K, the magnification of sweetness intensity was determined according to the following equation for calculating sweetness intensity.

$$Y = 19.09X^{0.424}$$

[Y: concentration equivalent to that of sucrose (g/100ml); X: concentration of Acesulfame K (g/100ml)]
For the sweetness intensity of the another sweetener with a high intense sweetness, the following magnifications were used.

Saccharine: 190 times, Sodium Saccharine: 190 times, disodium glycyrrhizinate: 100 times, Alitame: 2000 times, Glycyrrhizin: 170 times, Stevioside: 140 times, and Thaumatin: 850 times.

(Results of the sensory evaluation)

With respect to the characteristics of the taste, any one of those another sweetener with a high intense sweetness was very strong in bitter, lingering, and peculiar taste, and strong in irritation, astringent and heavy taste. It was also very weak in round and clear taste, and weak in the onset of the taste. However, by mixing these sweetener(s) with each derivatives 1 to 9, it was confirmed that the taste of each of derivatives 1 to 9 was improved and closer to the balanced taste of sucrose.

For example, though the aqueous solution and the cola drink having a sweetness level equivalent to that of PSE 10% in calculation can be prepared, the sensory evaluation thereof shows that the bitter, peculiar taste and so on are felt strongly, and they are different from that of PSE 10% as sweet taste. The total taste is not preferable. On the contrary, when derivative 1 was mixed with Acesulfame K in the sweetness ratio 5:5, for example, the similarity index is 1.00, when the ratio is 8:2, the similarity index is 1.02, and when the ratio is 9:1, the similarity index is 0.86. Thus, all the balance of the taste was improved, wherein the similarity index of Acesulfame K alone is closer without limit to 2.0, and that of sucrose is 0.

Next, with regard to the mixture of each of derivatives 1 to 9 with sodium cyclamate and suclarose, the sensory evaluation was performed as described above. The sweetener compositions having a sweetness level equivalent to that of PSE 10% were prepared in the following sweetness ratio.

| Derivatives 1 to 9 | : | Another sweetener with a high intense sweetness |
|---|---|---|
| 8 | : | 2 |
| 5 | : | 5 |
| 2 | : | 8 |

For the sweetness intensity of the another sweeteners with a high intense sweetness, were used such magnifications as sodium cyclamate: 30 times, and suclarose: 400 times.

(Results of the sensory evaluation)

In all cases, as the ratio of said sweetener with a .high intense sweetness became larger, the similarity index of any one of derivatives 1 to 9 became smaller compared to the taste of each of the derivatives alone and it was confirmed to be closer to that of sucrose.

(Example 6) Sweetener composition of derivative 1 and Aspartame

By using the equation for calculating sweetness intensity in the cola drink (refer to Example 4) and the equation for calculating sweetness intensity of Aspartame (refer to the monthly Journal "Food Chemical" August, page 35, 1997), the necessary amounts of those were calculated. In case of Aspartame, the values at 20°C, pH2.8 were used.

| Derivative 1 : Aspartame | Concentration of derivative 1(mg/1000ml) | Concentration of Aspartame (mg/1000ml) |
|---|---|---|
| 9.5 : 0.5 | 4.20 | 7.2 |
| 8 : 2 | 3.7 | 53 |
| 5 : 5 | 2.6 | 202 |
| 2 : 8 | 1.3 | 398 |

With regard to the 9 items of sweetness quality, the cola drinks of each sample having a sweetness level equivalent to that of PSE 10%, were compared with the cola drink of 10% sucrose for the sensory evaluation. The similarity index of each evaluation item was determined by 8 panelists according to Example 3.

(Results of the sensory evaluation)

When the ratio of sweetness intensity of derivative 1 was 95%, the early and round tastes are very weak. The similarity index is 0.76.

When the ratio of sweetness intensity of derivative 1 was 80%, the early and round tastes are very weak and clear taste is weak. The astringent, lingering, bitter and peculiar tastes are strong.
The similarity index is 0.74.

When the ratio of sweetness intensity of derivative 1 was 50%, the onset of the taste is very weak, and the round and clear tastes are weak. The astringent, lingering and bitter tastes are strong.
The similarity index is 0.64.

When the ratio of sweetness intensity of derivative 1 was 20%, the onset of the taste and round tastes are very weak.
The similarity index is 0.48.

As the similarity index of derivative 1 alone is 0.78, the sweet taste of the composition mixed with at least 5% Aspartame by the ratio of sweetness intensity, is found to be improved.

(Example 7) Sweetener composition of derivative 2 and Aspartame

The same experiment as Example 6 except for derivative 2 instead of derivative 1 in Example 6 was repeated.

With regard to derivative 2, for the equation for calculating sweetness intensity in the cola drink, that determined in Example 4 was used .

| Derivative 2 : Aspartame | Concentration of Derivative 2 (mg/ 1000ml) | Concentration of Aspartame (mg/1000ml) |
|---|---|---|
| 9.5 : 0.5 | 19 | 7.2 |
| 8 : 2 | 16 | 53 |
| 5 : 5 | 9.1 | 202 |
| 2 : 8 | 3.1 | 398 |

(Results of the sensory evaluation)

When the ratio of sweetness intensity of derivative 2 was 95%, the onset of the taste is very weak, and the round and clear tastes are weak. The lingering taste is very strong and the astringent and bitter tastes are strong.
The similarity index is 0.70.

When the ratio of sweetness intensity of derivative 2 was 80%, the early and round tastes are weak, and the astringent, lingering, and bitter tastes are strong.
The similarity index is 0.50.

When the ratio of sweetness intensity of derivative 2 was 50%, the early, round and clear tastes are weak, and the astringent, lingering, bitter and heavy tastes are strong.
The similarity index is 0.60.
When the ratio of sweetness intensity of derivative 2 was 20%, the onset of the taste is very weak, and the round and clear tastes are weak. The lingering taste is very strong, and the astringent and bitter tastes are strong.
The similarity index is 0.63.
As the similarity index of derivative 2 alone is 0.80, the sweet taste of the composition mixed with at least 5% Aspartame by sweetness intensity is found to be improved.

(Example 8) Sweetener composition of derivative 2 and Acesulfame K

The same experiment as Example. 7 except for Acesulfame K instead of Aspartame in Example 7 was repeated.
With regard to Acesulfame K, as the equation for calculating sweetness intensity in the cola drink, that determined in Example 5 was used.

| Derivative 2 : Acesulfame K | Concentration of Derivative 2 (mg/ 1000ml) | Concentration of Acesulfame K (mg/ 1000ml) |
|---|---|---|
| 9.5 : 0.5 | 4.2 | 1.9 |
| 9 : 1 | 4.1 | 9.5 |
| 8 : 2 | 3.7 | 48.9 |
| 5 : 5 | 2.6 | 424.4 |

(Results of the sensory evaluation)

When the ratio of sweetness intensity of derivative 2 was 95%, the onset of the taste and round tastes are weak. The astringent, lingering, bitter and peculiar tastes are strong.
The similarity index is 0.63.
When the ratio of sweetness intensity of derivative 2 was 90%, the onset of the taste, round and clear tastes are weak, and the bitter taste, irritation, astringent, lingering, peculiar and heavy tastes are strong.
The similarity index is 0.76.
When the ratio of sweetness intensity of derivative 2 was 80%, the round taste is very weak, and the onset of the taste and clear tastes are weak. The bitter taste is very weak, and the irritation, astringent, peculiar and lingering tastes are strong.
The similarity index is 0.77.
When the ratio of sweetness intensity of derivative 2 was 50%, the onset of the taste, round and clear tastes are weak. The astringent taste is very strong, and the irritation, astringent, peculiar, lingering and heavy tastes are strong.
The similarity index is 0.90.
As the similarity index of derivative 2 alone is 0.80, the sweet taste of the composition mixed with at most 20% Acesulfame K by the ratio of sweetness intensity is found to be improved.

(Example 9) Improvement of taste by salt

To the aqueous solution of derivative 1 having a sweetness level equivalent to that of PSE 10%, salt was added as final concentratins of 0.1%, 0.2% and 0.5% to compare with the aqueous solution of 10% sucrose for by 2 points comparison method (n=8) and evaluate the characteristics of the taste.
The equation for calculating sweetness intensity of derivative 1 in the aqueous solution is as follows.

$$Y = 6980X^{0.848}$$

| Sample No. | Concentration of derivative 1 (mg/100ml) | NaCl addition (%) | PSE(%) |
|---|---|---|---|
| 1 | 0.46 | 0.1 | 10 |

(continued)

| Sample No. | Concentration of derivative 1 (mg/100ml) | NaCl addition (%) | PSE(%) |
|---|---|---|---|
| 2 | 0.46 | 0.2 | 10 |
| 3 | 0.46 | 0.5 | 10.4 |

The sensory evaluation was performed by the same way as Example 6.

(Results of the sensory evaluation)

When salt was added in 0.1%, the onset of the taste, round and clear tastes are weak. The lingering, bitter, irritation, heavy and peculiar tastes are strong.
The similarity index is 0.77.

When salt was added in 0.2%, the onset of the taste and clear tastes are weak. The lingering, and peculiar tastes are very strong. The irritation and heavy taste are strong. The similarity index is 0.79.

When salt was added in 0.5%, the onset of the taste, and clear tastes are weak. The lingering and peculiar tastes, and irritation are very strong. The heavy taste is strong. The similarity index is 0.83.

As the similarity index of derivative 1 in the aqueous solution is 1.17 (refer to Example 2), the sweet taste of the composition by addition of salt is found to be improved. Other derivatives (derivatives 2 to 9) were evaluated by the same method as described above, and it was found that the addition of salt was effective and the amount of addition of salt not more than 0.3% by weight was preferable for improving the taste.

(Example 10) Use for the carbonated cola drink

The carbonated cola drink was produced in the following composition.

| Component | Product of the present invention*1 | Reference product 1 *2 | Reference product 2 |
|---|---|---|---|
| Sucrose | | | 100g |
| Derivative 2 | 9.1mg | 20.5mg | |
| Aspartame | 202mg | | |
| Citric acid (crystal) | 0.25g | 0.25g | 0.25g |
| Sodium citrate | 0.10g | 0.10g | 0.10g |
| 85% Phosphoric acid | 0.3g | 0.3g | 0.3g |
| Cola base | 2ml | 2ml | 2ml |
| Cola essence | 1ml | 1ml | 1ml |
| Distilled water | to1000ml*3 | to1000ml*3 | to1000ml*3 |

*1:Sweetness ratio 5:5, PSE 10%, pH2.8
*2:PSE 10% with derivative 2 alone, pH 2.8
*3:Addition of distilled water to final volume 1000 ml.

1000ml of each of the above-obtained cola drinks (the product of the present invention, the reference product 1 or the reference product 2) was charged into a carbonation bomb, and carbon dioxide gas was charged therein. The bomb was stored in the refrigerator for overnight. When it was cooled well, the bomb was opened while it stood still, and the solution thereof was immediately charged into 240ml can.

(Evaluation of the sweetness quality)

With respect to the products (PSE 10%) obtained by the method as described above, the sensory evaluation was performed by the same way. As a result, with respect to the reference product 1, the onset of the taste was very weak , the lingering taste was very strong, and the astringent taste was strong. On the contrary, with respect to the product of the present invention, the sweetness quality was improved such that the onset of the taste became stronger and the lingering and astringent tastes became weaker, the well-balanced taste characteristics were shown and also

the total taste became preferable (n=20).

(Example 11) Production of sweetener for tabletop use

The sweetener was produced by mixing well the following components.

| Component | Weight (**g**) | Composition (weight%) |
|---|---|---|
| Derivative 2 | 0.66 | 0.10 |
| Aspartame | 1.38 | 0.21 |
| Erythritol | 666.67 | 99.40 |
| Flavor | 1.97 | 0.29 |
| Total amount | 670.68 | 100 |

When 0.94 g of the sweetener produced (sample) were added to 140 ml (volume for the standard coffee cup) of coffee solution, the sweetness intensity of said coffee solution is equivalent to that of PSE 5%. The sweetness ratio of the sweetener is in derivative 2 : Aspartame : Erythritol = 4 : 0.5 : 0.5, and herein the magnification of sweetness intensity of the derivative 2 at PSE 4% was calculated as 6000 times, the magnification of sweetness intensity of Aspartame at PSE 0.5% was calculated as 360 times and the magnification of sweetness intensity of Erythritol even at PSE 0.5% was calculated as 0.75 times.

0.94g (/one cup[140ml]) of the above sweetener for tabletop use was added to coffee as a sweetener, and thus obtained coffee was compared to the coffee with 5g of sucrose (/one cup[140ml]) added by the sensory evaluation. There was no significant difference between the sweet tastes of the both, and the coffee using the above sweetener for tabletop use showed a similar sweetness (sweet taste) to that of sucrose with totally preferable taste (n=20) . And the above tabletop sweetener is superior in that the calorie of the sweetener is close to zero.

(Example 12) Production of a sherbet (ice block; block ice)

A sherbet (a block ice) is produced usually by freezing the aqueous solution of sucrose and flavors. Herein the sweetness of sucrose was replaced by that of the derivative used in the present invention. As the depression of freezing point of the derivative used in the present invention is smaller than that of sucrose, the freezing point thereof rose by 4 to 5°C. Therefore, it froze easily, it is difficult to prepare unevenness on freezing, and the surface thereof did not weep ("Naki" in Japanese was not found) . Further, it had a good long time stability for storage. When a fruit flavor was used, the product of good fruit juice flavor was obtained. Example: block ice (coffee type)

| Component | Composition (weight%) |
|---|---|
| Extract of coffee (Brix44.1) | 0.9 |
| Derivative 1 | 0.0002 |
| Aspartame | 0.038 |
| Deionized water | 99.0618 |
| Total amount | 100 |
| PH4.9; Bx.44.1 | |

The sweetness ratio of said block ice is derivative 1:Aspartame = 5:5, wherein the magnification of the sweetness intensity of derivative 1 at PSE 5% was calculated as 25600 times, and that of Aspartame at PSE 5% was calculated as 130 times.

(Example 13) Production of an orangeade

The orangeades were produced in the following compositions.

| Component | Product of the present invention*1 | Reference product*2 |
|---|---|---|
| Concentrated orange juice*3 | 17.5g | 17.5g |
| Liquid sugar of fructose and glucose (Intense sweetness 1) | 18.0g | 18.0g |
| PO-40*4 | 27.7g | 27.7g |
| Derivative 4 | 0.00012g | 0.00015g |
| Aspartame | 0.0506g | - |
| Citric acid | 3g | 3g |
| Vitamin C | 0.2g | 0.2g |
| Distilled water | To1000g | to1000g |

*1:Total sweetness ratio of sweetness intensity of total product; derivative 4 : Aspartame : sweetness derived from concentrated fruit juice: sweetness derived from the liquid sugar of fructose and glucose : sweetness derived from PO-40 = 5.1 :1.2 : 1.1 : 1.8 : 0.8.

The sweetness ratio between two types of the sweetener with a high intense sweetness; derivative 4 : Aspartame = 8:2.

*2: Total sweetness ratio; derivative 4 : sweetness derived from concentrated fruit juice: sweetness derived from liquid sugar of fructose and glucose : sweetness derived from PO-40 = 6.3:1.1:1.8:0.8.

*3: IRF 1/5.7 (intensity of sugar 63.6)

*4: Towa Kasei Co. Ltd., Reduced starch sugar, solid matter 70% (intensity of sweetness 0.4).

Both products of the present invention and the reference for the above orangeade contain 10% fruit juice, 10% intensity of sweetness (PSE), 16.5 Kcal total energy (they can be labeled as "low-calorie" because it is not more than 20 Kcal which is a standard of showing nutrition therefor), and 2.46g/100g sugar (they can be labeled as "low-sugar" because it is not more than 2.5g/100g which is a standard of showing nutrition therefor).

Where, the calculation of the sweetness was made by using the equation for calculating sweetness intensity of derivative 4 $Y=58775X^{1.04}$. When Y=6.3%, X=0.00015g/100ml, When Y=5.1%, X=0.00012g/100ml.

(Evaluation of the sweetness quality)

As described above, the sensory evaluation for the products obtained (PSE 10%) was performed. As a result, the sweetness quality of the product of the present invention was improved in comparison with that of the reference product such that the onset of the taste became stronger, the astringent and bitter tastes became weaker, and the well-balanced taste characteristics was shown and the total taste was preferable (n=20).

(Second invention of the present invention)

(Example 14) Measurement of the magnification of sweetness intensity

An aqueous solution was prepared by diluting derivative 2 to be PSE 10% concentration (15.5 mg/1000 ml = 10/6500 g/100 ml), assuming that the intensity of sweetness of derivative 2 was 6500 times that of sucrose. Separately, aqueous sucrose solutions having sucrose concentrations of (a)6.94%, (b)8.33%, (c)10%, (d)12%, and (e)14.4% were prepared. The sensory evaluation was performed by determining which sucrose solution was closest to the solution of derivative 2 in the sweetness intensity. The result of calculation of the average of points of 20 panelists was 2.25 point.

The intensity of sweetness of the solution of derivative 2 was 8.75% according to the following equation: (10.0-8.33) x 0.25 +8.33=8.75. Accordingly, the intensity of sweetness of derivative 2 was 5600 (=8.75/0.00155) times that of sucrose. According to the same experiment, the intensity of sweetness of derivative 1 was 22600 times that of sucrose. Furthermore, the magnification of sweetness intensity of other derivatives (3 to 9) can be determined by the same method.

And the magnification of sweetness intensity in the cola drink can be also determined by the same method compared to the reference solution of cola drink containing 10% sucrose.

The components of cola drink is as follows.

| | |
|---|---|
| Citric acid (crystal) | 0.25g/1000ml |
| Sodium citrate | 0.10g/1000ml |

(continued)

| | |
|---|---|
| 85% Phosphoric acid | 0.3g/1000ml |
| Cola base | 2ml/1000ml |
| Cola essence | 1ml/1000ml |
| Sweetener(sample) | Prescribed amount |

As for the concentration of the references, the sucrose concentrations of previous (a) to (e) were used. As the results, the magnification of sweetness intensity in the cola drink of derivative 1 was 22600 times, and that of derivative 2 was 4900 times.

(Example 15) The taste characteristics (In aqueous solution)

The following experiments were conducted using the water obtained by ion exchanging and further distilling tap water.

An aqueous solution of each of derivatives 1 to 9 having a sweetness level equivalent to that of PES 10% was prepared, and compared with aqueous 10%sucrose solution with respect to 9 items, namely, "the onset of the taste", "round taste", "clear taste", "lingering taste", "peculiar taste", "heavy taste", "bitter taste", "astringent taste" and "irritation". The results were determined as 5 levels (-2 point: very weak, -1 point: a little weak, 0 point: same, +1 point: a little strong, +2 point: very strong) by 8 panelists, and the average points were calculated. Results were as follows.

| Sample | Amount equivalent to PSE 10% (mg/1000ml) |
|---|---|
| Derivative 1 | 4.5 |
| Derivative 2 | 17.9 |
| Derivative 3 | 2.3 |
| Derivative 4 | 2.3 |
| Derivative 5 | 11.9 |
| Derivative 6 | 6.7 |
| Derivative 7 | 9.0 |
| Derivative 8 | 5.5 |
| Derivative 9 | 12.5 |

(Results of the taste characteristics)

All of the derivatives were extremely weak in the onset of the taste, very weak in round and clear taste, extremely strong in lingering taste, and very strong in peculiar, heavy, bitter and astringent taste, and irritation.

Next, the total of the absolute value of the deviation from sucrose in each of the evaluation items consisting of onset of the taste, round taste, clear taste, lingering taste, peculiar taste, heavy taste, bitter taste, astringent taste and irritation was divided by 9 (total number of items) to calculate the similarity (which is refer to as "similarity index"). The smaller the similarity index becomes, the better the taste balances, and the closer the taste is to the taste characteristics (quality) of sucrose. The total taste becomes preferable. The similarity indexs of each of the derivatives are shown as follows.

| Sample | Similarity index |
|---|---|
| Derivative 1 | 1.17 |
| Derivative 2 | 0.63 |
| Derivative 3 | 1.04 |
| Derivative 4 | 0.93 |
| Derivative 5 | 1.38 |

(continued)

| Sample | Similarity index |
|---|---|
| Derivative 6 | 0.93 |
| Derivative 7 | 0.86 |
| Derivative 8 | 0.77 |
| Derivative 9 | 0.77 |

(Example 16) The taste characteristics (in cola drink)

The following experiments were conducted in the same way as that of Example 15. Instead of aqueous solution, the cola drink of each of derivatives 1 to 9 having a sweetness level equivalent to that of PSE 10% was prepared, and compared with the cola drink containing 10% sucrose. All of the evaluation method and so on were same as those of Example 15.

When carbon dioxide is blown in the cola drink, the carbonated cola drink is prepared, however, the taste of the cola drink can be compared more easily in that without carbon dioxide. Thus the non-carbonated cola drink was organoleptically evaluated.

The composition of the cola drink employed was same as that of Example 14.

| Sample | Amount equivalent to PSE 10% (mg/1000ml) |
|---|---|
| Derivative 1 | 4.5 |
| Derivative 2 | 20.4 |
| Derivative 3 | 2.7 |
| Derivative 4 | 3.4 |
| Derivative 5 | 12.5 |
| Derivative 6 | 7.1 |
| Derivative 7 | 9.4 |
| Derivative 8 | 6.3 |
| Derivative 9 | 13.3 |

(Results of the taste characteristics)

All of the derivatives were extremely weak in the onset of the taste, very weak in round and clear taste, extremely strong in lingering taste, and very strong in peculiar, heavy, bitter and astringent taste, and irritation.

The similarity indexes were calculated as those of aqueous solutions. The smaller the similarity index becomes, the better the taste balances, and the closer the taste is to the taste characteristics (quality)of sucrose. The total taste becomes preferable. The similarity indexes of each of the derivatives are shown as follows.

| Sample | 'Similarity index |
|---|---|
| Derivative 1 | 0.78 |
| Derivative 2 | 0.80 |
| Derivative 3 | 1.11 |
| Derivative 4 | 1.04 |
| Derivative 5 | 1.34 |
| Derivative 6 | 0.81 |
| Derivative 7 | 0.90 |
| Derivative 8 | 1.00 |

(continued)

| Sample | 'Similarity index |
|---|---|
| Derivative 9 | 1.00 |

(Example 17) Improvement of taste by sugar and so on used in the present invention

The mixture of each sample of derivatives 1 to 9 with sugar and so on used in the present invention was prepared and the sensory evaluation was performed by comparing with the cola drink containing 10% sucrose concentration in the same manner as the method described in Example 16.

The sweetener composition with the sugar and so on equivalent to the sweetness level of PSE 10% was prepared in the following sweetness ratio.

| Derivatives 1 to 9 : Sugar and so on | | |
|---|---|---|
| 8 | : | 2 |
| 5 | : | 5 |
| 2 | : | 8 |

Examples for equations for calculating sweetness intensity in the cola drink (pH2.8, 20°C) are as follows, and another equations can be prepared in the same manner, wherein Y denotes the concentration equivalent to that of sucrose (g/100ml) and X denotes the concentration of the sweetener (g/100ml), respectively.

| Sample | Equations for calculating sweetness intensity |
|---|---|
| Derivative 1 | $Y = 212300 \times X^{1.29}$ |
| Derivative 2 | $Y = 1890 \times X^{0.847}$ |
| Derivative 6 | $Y = 9520 \times X^{0.947}$ |
| Derivative 8 | $Y = 3940 \times X^{0.812}$ |

As the sugar and so on used in the above present invention, at least one of the following compounds can be used, and the following numerical values put in the brackets were used as the magnification of sweetness intensity of those compounds.

Sugar: Sucrose (1), invert sugar (1), isomerized sugar (1), glucose (0.6), fructose (1.4), lactose (0.2), malt sugar (0.3), D-xylose (0.4) and isomerized lactose (0.6).

Sugar alcohol: maltitol (reduced maltose syrup) (0.75), sorbitol (0.75), mannitol (0.6), erythritol (0.75), xylitol (1), lactitol (reduced lactose) (0.35), paratinit (0.45), and reduced starch sugar (hydrogenated starch syrup) (0.5).

Oligosaccharide: fructooligosaccharide (neosugar) (0.5), maltooligosaccharide (linear chain oligosaccharide) (0.33), isomaltooligosaccharide (branched chain oligosaccharide) (0.5), galactooligosaccharide (0.2), soy been oligosaccharide (0.7) and lactooligosaccharide (0.8). A derivative of sucrose: sucrose binding starch sugar (coupling sugar: glucosylsucrose) (0.5), paratinose (isomaltulose) (0.4) and trehalose (0.45).

(Results of the sensory evaluation)

In all cases, as the ratio of sweetness intensity of the sugar and so on used in the present invention became larger, the similarity index became smaller compared to the result of each of derivatives 1 to 9 alone (refer to Example 16) and it was confirmed that the taste becomes closer to that of sucrose.

In addition, the another sugar and so on which is not exemplified above (sugar and so on used in the present invention) may show the same effect by doing the same experiment, if preformed.

(Example 18) Sweetener composition of derivative 1 and sucrose

With respect to the sweetener compositions of derivative 1 and sucrose the sensory evaluation was performed

by comparing with the cola drink with sucrose dissolved therein in a 10% concentration according to the method described in Example 16.

Those sweetener compositions equivalent to the sweetness level of PSE 10% were prepared in the following sweetness ratio. The amount necessary for derivative 1 was calculated by using the equation for calculating sweetness intensity in the cola drink (refer to Example 17).

| Derivative 1 : Sucrose (Sweetness ratio) | Concentration of derivative 1 | Concentration of Sucrose |
|---|---|---|
| | (mg/1000ml) | (mg/1000ml) |
| 9.5 : 0.5 | 4.3 | 5 |
| 8 : 2 | 3.7 | 20 |
| 5 : 5 | 2.6 | 50 |
| 2 : 8 | 1.3 | 80 |
| 0.5 : 9.5 | 0.4 | 95 |

With regard to the 9 items of sweetness quality described above, the cola drinks with each sample having a sweetness level equivalent to that of PSE 10%, were compared with the cola drinks with 10% sucrose for the sensory evaluation. The similarity index of each item of evaluation was determined by 8 panelists according to Example 16.

(Results of the sensory evaluation)

The similarity index of derivative 1 alone is 0.78, and however, by mixing therewith sucrose, the sweet tastes of the compositions thus obtained such as the onset of the taste and lingering taste were improved, and the effect of the improvement became much more as the sweetness ratio of sucrose became larger.

| Derivative 1 : Sucrose (Sweetness ratio) | Similarity index |
|---|---|
| 9.5 : 0.5 | 0.75 |
| 8 : 2 | 0.73 |
| 5 : 5 | 0.66 |
| 2 : 8 | 0.51 |
| 0.5 : 9.5 | 0.35 |

Next, the relationship between the sweetness ratio and the weight ratio of derivative 1 and sucrose and so on are shown.

It can be calculated by using the equation for calculating sweetness intensity on the derivative 1 (refer to Example 17).

When the sweetness ratio is derivative 1:sucrose, lactose and so on = 0.5:9.5,
derivative 1 of PSE 0.5% :0.0000434g/dl;
sucrose and so on of PSE 9.5% (intensity of sweetness 1) :9.5g/dl; and
lactose and so on of PSE 9.5% (intensity of sweetness 0.2):9.5/0.2g/dl.

Therefore, the weight% of derivative 1 in the mixture is in the following:

(a) In case of mixture with sucrose and so on (intensity of sweetness 1),

$$4.6ppm = 100 \times (0.0000434g/dl)/(0.0000434g/dl+9.5g/dl).$$

(b) In case of mixture with lactose and so on (intensity of sweetness 0.2),

$$0.9ppm = 100 \times (0.0000434g/dl)/(0.0000434g/dl+47.5g/dl)$$

When the sweetness ratio is derivative 1:sucrose, lactose and so on = 2:8,

in case of mixture with sucrose and so on (intensity of sweetness 1);the ratio (weight, ppm) of derivative 1 in the mixture is as follows.

| Derivative 1 : Sucrose and so on (Sweetness ratio) | Ratio of derivative 1 by weight in the mixture (ppm) |
|---|---|
| 2 : 8 | 16 |
| 8 : 2 | 185 |
| 9.5 : 0.5 | 849 |

Therefore, it is understood that they may be mixed suitably in the range of the weight ratio (ppm) of derivative 1 in the mixture of from 0.9ppm considering the case of low intense sweetness such as that of lactose and so on (intensity of sweetness 0.2), to 849ppm in case of 95% of the sweetness intensity when mixing with sucrose and so on (intensity of sweetness 1), and preferably, from 1 to 900ppm.
Next, the relationship between the sweetness ratio and the weight ratio of derivative 2 and sucrose and so on are shown.
It can be calculated by using the equation for calculating sweetness intensity on the derivative 2 (refer to Example 17).
When the sweetness ratio is derivative 2:sucrose, lactose and so on = 0.5:9.5,
Derivative 2 of PSE 0.5% 1:0.0000597g/dl;
sucrose and so on of PSE 9.5% (intensity of sweetness 1) :9.5g/dl; and
lactose and so on of PSE 9.5% (intensity of sweetness 0.2):9.5/0.2g/dl.
The weight ratio of derivative 2 in the mixture with lactose and so on (intensity of sweetness 0.2) mixtured was 1.26ppm.
When the sweetness ratio is derivative 2:sucrose, lactose and so on = 9.5:0.5,

the weight ratio of derivative 2 in the mixture with sucrose and so on (intensity of sweetness 1) mixed was 3850ppm.

Therefore, it is understood that they may be mixed in the range of the weight ratio of derivative 2 in the mixture of from 1.26 to 3850ppm, and preferably, from 1 to 4000ppm, as determined above.
When they are thus calculated for all of the derivatives used in the present invention, the weight ratio (value) applicable to all the derivatives for the present invention is in the range of 0.5 to 5000ppm.

(Example 19) Sweetener composition of derivative 2 and sucrose

With respect to the sweetener compositions of derivative 2 and sucrose the sensory evaluation was performed by comparing with the cola drink with sucrose dissolved therein in a 10% concentration according to the method described in Example 16.
Those sweetener compositions equivalent to the sweetness level of PSE 10% were prepared in the following sweetness ratio. The amount necessary for derivative 2 was calculated by using the equation for calculating sweetness intensity in the cola drink (refer to Example 17).

| Derivative 2 : Sucrose (Sweetness ratio) | Concentration of derivative 2 (mg/ 1000ml) | Concentration of Sucrose (mg/ 1000ml) |
|---|---|---|
| 9.5 : 0.5 | 19.3 | 5 |
| 8 : 2 | 15.8 | 20 |
| 5 : 5 | 9.1 | 50 |
| 2 : 8 | 3.1 | 80 |
| 0.5 : 9.5 | 0.5 | 95 |

With regard to the 9 items of sweetness quality described above, the cola drinks with each sample having a sweetness level equivalent to that of PSE 10%, were compared with the cola drinks with 10% sucrose for the sensory evaluation. The similarity index for each item of evaluation was determined by 8 panelists according to Example 16.

(Results of the sensory evaluation)

The similarity index of derivative 2 alone is 0.80, and however, by mixing therewith sucrose, the sweet taste of the compositions thus obtained such as the onset of the taste and lingering taste were improved, and the effect of the improvement became much more as the sweetness ratio of sucrose became larger.

| Derivative 2 : Sucrose (Sweetness ratio) | Similarity index |
|---|---|
| 9.5 : 0.5 | 0.75 |
| 8 : 2 | 0.42 |
| 5 : 5 | 0.46 |
| 2 : 8 | 0.31 |
| 0.5 : 9.5 | 0.20 |

(Example 20) Sweetener composition of derivative 1 and erythritol

With respect to the sweetener compositions of derivative 1 and erythritol the sensory evaluation was performed by comparing with the cola drink with sucrose dissolved therein in a 10% concentration according to the method described in Example 16.

Those sweetener compositions equivalent to the sweetness level of PSE 10% were prepared in the following sweetness ratio. The amounts necessary for derivative 1 were calculated by using the equation for calculating sweetness intensity in the cola drink (refer to Example 17).

| Derivative 1 : Erythritol (Sweetness ratio) | Concentration of derivative 1 (mg/ 1000ml) | Concentration of Erythritol (mg/ 1000ml) |
|---|---|---|
| 9.5 : 0.5 | 4.3 | 6.7 |
| 8 : 2 | 3.7 | 26.7 |
| 5 : 5 | 2.6 | 66.7 |
| 2 : 8 | 1.3 | 106.7 |
| 0.5 : 9.5 | 0.4 | 126.7 |

With regard to the 9 items of sweetness quality described above, the cola drinks with each sample having a sweetness level equivalent to that of PSE 10%, were compared with the cola drinks with 10% sucrose for the sensory evaluation. The similarity index for each item of evaluation was determined by 8 panelists according to Example 16.

(Results of the sensory evaluation)

The similarity index of derivative 1 alone is 0.78, and however, by mixing therewith erythritol, the sweet taste of the compositions thus obtained such as the onset of the taste, lingering and bitter tastes were improved, and the effect of the improvement became much more as the sweetness ratio of erythritol became larger.

| Derivative 1 : Erythritol (Sweetness ratio) | Similarity index |
|---|---|
| 9.5 : 0.5 | 0.75 |
| 8 : 2 | 0.74 |
| 5 : 5 | 0.72 |
| 2 : 8 | 0.35 |
| 0.5 : 9.5 | 0.30 |

(Example 21) Sweetener composition of derivative 2 and erythritol

With respect to the sweetener compositions of derivative 2 and erythritol the sensory evaluation was performed by comparing with the cola drink with sucrose dissolved therein in a 10% concentration according to the method described in Example 16.

Those sweetener compositions equivalent to the sweetness level of PSE 10% were prepared in the following sweetness ratio. The amounts necessary for derivative 2 were calculated by using the equation for calculating sweetness intensity in the cola drink (refer to Example 17).

| Derivative 2 : Erythritol (Sweetness ratio) | Concentration of derivative 2 (mg/ 1000ml) | Concentartion of Erythritol (mg/ 1000ml) |
|---|---|---|
| 9.5 : 0.5 | 19.3 | 6.7 |
| 8 : 2 | 15.8 | 26.7 |
| 5 : 5 | 9.1 | 66.7 |
| 2 : 8 | 3.1 | 106.7 |
| 0.5 : 9.5 | 0.5 | 126.7 |

With regard to the 9 items of sweetness quality described above, the cola drinks with each sample having a sweetness level equivalent to that of PSE 10%, were compared with the cola drinks with 10% sucrose for the sensory evaluation. The similarity index for each item of evaluation was determined by 8 panelists according to Example 16.

(Results of the sensory evaluation)

The similarity index of derivative 2 alone is 0.80, and however, by mixing therewith erythritol, the sweet taste of the compositions thus obtained such as the onset of the taste and lingering taste were improved, and the effect of the improvement became much more as the sweetness ratio of erythritol became larger.

| Derivative 2 : Erythritol (Sweetness ratio) | Similarity index |
|---|---|
| 9.5 : 0.5 | 0.65 |
| 8 : 2 | 0.60 |
| 5 : 5 | 0.46 |
| 2 : 8 | 0.30 |
| 0.5 : 9.5 | 0.25 |

(Example 22) Use for a carbonated cola (No.1)

The carbonated cola drinks were produced in the following compositions.

| Component | Product of the present invention*1 | Reference product 1*2 | Reference product 2 |
|---|---|---|---|
| Derivative 1 | 2.6mg | 4.4mg | |
| Sucrose | 50g | | 100g |
| Citric acid (crystaline) | 0.25g | 0.25g | 0.25g |
| Sodium citrate | 0.10g | 0.10g | 0.10g |
| 85% Phosphoric acid | 0.3g | 0.3g | 0.3g |
| Cola base | 2ml | 2ml | 2ml |
| Cola essence | 1ml | 1ml | 1ml |
| Distilled water | to1000ml*3 | to1000ml*3 | to1000ml*3 |

*1: Sweetness ratio 5:5 (derivative 1 : sucrose), PSE 10%, pH2.8;
*2: PSE 10% with derivative 1 alone, , pH 2.8;
*3: Addition of distilled water to final volume 1000 ml.

1000ml of each of the above-obtained cola drinks (the product of the present invention, the reference product

1 or the reference product 2) was charged into a carbonation bomb, and carbon dioxide gas was charged therein. The bomb was stored in the refrigerator for overnight. When it was cooled well, the bomb was opened while it stood still, and the solution thereof was immediately charged into 240ml can.

(Evaluation of sweetness quality)

With respect to the three products obtained above (PSE 10%), the sensory evaluation in terms of the 9 items described above (the onset of the taste, the peculiar taste, the heavy taste, the irritation, the bitter taste, the lingering taste, a stringent taste, the clear taste and the round taste) was performed in the same way. As a result, the reference product 1 ( use of derivative 2 alone)was very weak in the onset of the taste, very strong in the lingering taste, and strong in the astringent taste. On the contrary, the product of the present invention was improved in the sweetness quality such that the onset of the taste became stronger and the lingering and astringent tastes became weaker, and thereby the well-balanced taste characteristics was shown and the total taste thereof was preferable (n=20).

(Example 23) Use for a carbonated cola (No.2)

The carbonated cola drinks were produced in the following compositions.

| Component | Product of the present invention*1 | Reference product 1*2 | Reference product 2 |
|---|---|---|---|
| Derivative 2 | 15.8mg | 20.5mg | |
| Sucrose | | | 100g |
| Erythritol | 26.7mg | | |
| Citric acid (crystaline) | 0.25g | 0.25g | 0.25g |
| Sodium citrate | 0.10g | 0.10g | 0.10g |
| 85% Phosphoric acid | 0.3g | 0.3g | 0.3g |
| Cola base | 2ml | 2ml | 2ml |
| Cola essence | 1ml | 1ml | 1ml |
| Distilled water | to1000ml*3 | to1000ml*3 | to1000ml*3 |

*1: Sweetness ratio 8:2 (derivative 1:Erythritol), PSE 10%, pH2.8;
*2: PSE 10% with derivative 2 alone, pH 2.8;
*3: Addition of distilled water to final volume 1000 ml.

1000ml of each of the above-obtained cola drinks (the product of the present invention, the reference product 1 or the reference product 2) was charged into a carbonation bomb, and carbon dioxide gas was charged therein. The bomb was stored in the refrigerator for overnight. When it was cooled well, the bomb was opened while it stood still, and the solution thereof was immediately charged into 240ml can.

(Evaluation of sweetness quality)

With respect to the three products obtained above (PSE 10%), the sensory evaluation in terms of the 9 items described above, was performed in the same way. As a result, the reference product 1 ( use of derivative 2 alone) was very weak in the onset of the taste, very strong in the lingering taste, and strong in the astringent taste. On the contrary, the product of the present invention was improved in the sweetness quality such that the onset of the taste became stronger, and the lingering and astringent tastes became weaker, and thereby the well-balanced taste characteristics were shown and the total taste thereof was preferable (n=20).

(Example 24) Use for a carbonated cola (No.3)

The carbonated cola drinks were produced in the following compositions.

| Component | Product of the present invention*1 | Reference product 1*2 | Reference product 2*3 | Reference product |
|---|---|---|---|---|
| Derivative 1 | 2.2mg | 4.4mg | | |
| Derivative 2 | 7.0mg | | 20.5mg | |
| Sucrose | 10g | | | 100g |
| Erythritol | 13.3g | | | |
| Citric acid (crystaline) | 0.25g | 0.25g | 0.25g | 0.25g |
| Sodium citrate | 0.10g | 0.10g | 0.10g | 0.10g |
| 85% Phosphoric acid | 0.3g | 0.3g | 0.3g | 0.3g |
| Cola base | 2ml | 2ml | 2ml | 2ml |
| Cola essence | 1ml | 1ml | 1ml | 1ml |
| Distilled water | to1000ml*4 | to1000ml*4 | to1000ml*4 | to1000ml *4 |

*1: Sweetness ratio, derivative 1:derivative 2:sucrose:Erythritol=4:4:1:1, PSE 10%, pH2.8;

*2: PSE 10% with derivative 1 alone, pH 2.8;

*3: PSE 10% with derivative 2 alone, pH 2.8;

*4 :Addition of distilled water was added to final volume 1000 ml.

1000ml of each of the above-obtained cola drinks (the product of the present invention, the reference products 1 to 3) was charged into a carbonation bomb, and carbon dioxide gas was charged thereinto. The bomb was stored in the refrigerator for overnight. When it was cooled well, the bomb was opened while it stood still, and the solution thereof was immediately charged into 240ml can.

(Evaluation of sweetness quality)

With respect to the four products obtained above(PSE 10%), the sensory evaluation in terms of the 9 items described above was performed for mutual comparison in the same manner. As a result, compared with the reference product 1 ( use of derivative 1 alone) and the reference product 2 (use of derivative 2 alone), the product of the present invention (the mixture of derivative 1, derivative 2, erythritol and sucrose) was improved in the sweetness quality such that the onset of the taste became stronger, and the lingering and astringent tastes became weaker, and thereby the well-balanced taste characteristics was shown and the total taste thereof was preferable (n=20).

(Example 25) Production of sweetener for tabletop use

The sweetener was produced by mixing the following components sufficiently.

| Component | Weight | Composition (parts per million, ppm) |
|---|---|---|
| Derivative 2 | 0.70g | 700ppm |
| Sucrose | 1000g | |
| Total amount | 1000.7g | |

When 1.4 g of the sample obtained were added to 140 ml (volume for the standard coffee cup) of coffee solution, the sweetness intensity of said coffee solution is equivalent to that of PSE 5% . The sweetness ratio of the sweetener is in derivative 2:Sucrose = 4:1, and herein the magnification of sweetness intensity of the derivative 2 at PSE 4% was calculated as 6000 times.

1.4g (/one cup[140ml]) of the above sweetener for tabletop use was added to coffee, and the coffee was compared to thus obtained coffee with 5g of sucrose (/one cup[140ml]) added by the sensory evaluation. There was no significant difference between the sweet tastes of the both, and the coffee using the above sweetener for tabletop use showed a sweetness (sweet taste) similar to that of sucrose with totally preferable taste (n=20).

(Example 26) Production of a grapeade

The grapeades were produced in the following compositions.

| Component | Product of the present invention*1 | Reference product*2 |
|---|---|---|
| Derivative 8 | 3.6mg | 5.5mg |
| Concentrated grape juice*3 | 16.7g | 16.7g |
| liquid sygar of fructose and glucose*4 | 17.9g | - |
| PO-40*5 | 28.3g | - |
| Citric acid | 1g | 1g |
| Sodium citrate | 0.3g | 0.3g |
| DL-malic acid | 1.2g | 1.2g |
| Flavor | 1g | 1g |
| Distilled water | to1000g | to1000g |

*1:Total sweetness ratio; derivative 8:sweetness derived from the concentrated fruit juice:sweetness derived from the liquid sugar of fructose and glucose:sweetness derived from PO-40=6.3:1.1:1.8:0.8. The sweetness ratio between derivative 8 and other sweetener with a low intense sweetness is in the ratio of 6.3:3.7.

*2: Total sweetness ratio; derivative 8:sweetness derived from the concentrated fruit juice=8.9:1.1

*3: IRF1/6 (intense sugar 66.9)

*4: intensity of sweetness 1

*5: Reduced starch sugar produced by Towa Kasei Co. Ltd., solid component 70%, intensity of sweetness 0.4.

Both products of the present invention and the reference for the above grapeade contain 10% fruit juice, 10% intensity of sweetness (PSE), 16.3 Kcal/100ml total energy (they can be labeled as "low-calorie" because it is not more than 20 Kcal which is the standard of showing nutrition therefor), and 2.46g/100ml sugar (they can be labeled as "low-sugar" because it is lower than 2.5g/100ml which is a standard of showing nutrition therefor).

Incidentally, the calculation of the sweetness was made by using the equation for calculating sweetness intensity of derivative 8 (refer to Example 17). When Y=6.3%, X=0.00036g/100ml, and when Y=8.9%, X=0.00055g/100ml.

(Evaluation of sweetness quality)

With respect to the products (PSE 10%) obtained as described above, the sensory evaluation was performed. As a result, compared with the reference product, the product of the present invention was improved in the sweetness quality such that the onset of the taste became stronger, the lingering taste became weaker, and the astringent and bitter tastes became weaker, and thereby the well-balanced taste characteristics was shown and the total taste thereof was preferable (n=20).

(Example 27) Production of an ice candy

An ice candy is produced by freezing the aqueous solution of sucrose, fruit juice, thickening agent and flavor. Herein the sweetness (sweet taste) of sucrose was replaced by that of the aspartyl dipeptide ester derivative used in the present invention. As the depression of freezing point of the derivative used in the present invention is smaller than that of sucrose, the freezing point thereof rose by 4 to 5°C. Therefore, it froze easily, it is difficult to prepare without unevenness from freezing, and the surface thereof did not weep ("Naki" in Japanese was not found.). Further, it had a good long time stability for storage. When a fruit flavor was used, the product of good fruit juice flavor was obtained.

The ice candies were produced in the following compositions.

| Component | Product of the present invention*1 | Reference product*2 |
|---|---|---|
| Derivative 6 | 6.9mg | - |

*1:Total sweetness ratio; derivative 6:sweetness derived from the liquid sugar of fructose and glucose:sweetness derived from concentrated fruit juice (PSE 20.5%)=4.7:4.7:0.6.

*2: Total intensity of sweetness:PSE 20.5%

(continued)

| Component | Product of the present invention*1 | Reference product*2 |
|---|---|---|
| Sucrose | - | 96.0g |
| Liquid sugar of fructose and glucose*3 | 96.0g | 96.0g |
| Tartaric acid | 0.5g | 0.5g |
| Concentrated orange juice (1/5) | 19.7g | 19.7g |
| Xanthan gum | 0.5g | 0.5g |
| Carrageenan | 0.5g | 0.5g |
| Locust bean gum | 0.5g | 0.5g |
| Flavor | 1.5g | 1.5g |
| Distilled water | to1000g | to1000g |

*1:Total sweetness ratio; derivative 6:sweetness derived from the liquid sugar of fructose and glucose:sweetness derived from concentrated fruit juice (PSE 20.5%)=4.7:4.7:0.6.

*2: Total intensity of sweetness:PSE 20.5%

*3: Intensity of sweetness 1

*4: IRF1/5.7 (intense sugar 63.6)

Incidentally, the calculation of the sweetness was made by using the equation for calculating sweetness intensity of derivative 6 (refer to Example 17). When Y=9.6%, X=0.000685g/100ml.

(Evaluation of sweetness quality)

The product of the present invention and the reference product were compared by the sensory evaluation. There was no significant difference between the both sweetnesses thereof, and the product of the present invention gave a preferable taste similar to that of sucrose with totally preferable taste(n=20).

(Third invention of the present invention)

(Example 28)

With respect to the solution produced by adding derivative 1 (the product of the present invention), or adding nothing (no addition; the reference product) in the concentration shown in the following table 4 to the amino acid solution (2% solution of arginine by weight), the sensory evaluation was performed (using 10 panelists), and the equivalent concentration of bitter taste was determined. The results were shown in the table 4, wherein the equivalent concentration of bitter taste was shown in the concentration of aqueous solution of anhydrous caffeine which is a substance of bitter taste.

[Table 4]

| Sample | Amount of addition (ppm) | Equivalent concentration of bitter taste (g/100g) |
|---|---|---|
| No addition | - | 0.131 |
| Derivative 1 | 4.4 | 0.060 |

(Example 29)

With respect to the solution produced by adding derivative 2, Glycyrrhizin or sucrose (the product of the present invention), or adding nothing (no addition; the reference product) to 2% (by weight) solution of the mixed amino acids (leucine:valine:isoleucine=1:0.5:0.5 by weight), the sensory evaluation was performed (using 10 panelists), and the equivalent concentration of bitter taste was determined in the same way as that described in Example 28, and shown in the table 5.

[Table 5]

| Sample | Amount of addition (ppm) | Equivalent concentration of bitter taste (g/100g) |
|---|---|---|
| No addition | - | 0.105 |
| Derivative 2 | 10 | 0.051 |
| Glycyrrhizin | 250 | 0.105 |
| Sucrose | 50000 | 0.067 |

(Example 30)

With respect to the solution produced by adding derivative 4 (2.2 ppm) to each of the substances of Quinine sulfate (39ppm), anhydrous caffeine (2000ppm), ferrous citrate (50ppm), calcium lactate (1500ppm), and thiamine hydrochloride (5000ppm) (the product of the present invention), or adding nothing thereto (no addition; the reference product) the reference product (no addition), the sensory evaluation was performed (using 10 panelists), and the equivalent concentration of bitter taste was determined in the same way as that described in Example 28, and shown in table 6.

[Table 6]

| Equivalent concentration of bitter taste (Amount of anhydrous caffeine g/100g) | | | | | |
|---|---|---|---|---|---|
| Sample | Substance of bitter taste | | | | |
| Component | Quinine sulfate | caffeine (anhydrous) | Ferrous citarte | Calcium lactate | Tiamine hydrochl oride |
| No addition | 0.084 | 0.205 | 0.027 | 0.054 | 0.131 |
| Derivative 4 | 0.025 | 0.045 | 0.020 | 0.025 | 0.093 |

(Example 31) Measurement of the magnification of sweetness intensity

For the aspartyl dipeptide ester derivative used in the present invention one having a high intense sweetness, and particularly one having a intense sweetness not less than 4000 times that of sucrose are preferable. Thus, the method of measuring the magnification of sweetness is sown as follows.

An aqueous solution was prepared by diluting derivative 2 to be PSE 10% concentration (15.5 mg/1000 ml = 10/6500 g/100 ml), assuming that the intense sweetness of derivative 2 was 6500 times that of sucrose. Separately, aqueous sucrose solutions having sucrose concentrations of (a)6.94%, (b)8.33%, (c)10%, (d)12%, and (e)14.4% were prepared. The sensory evaluation was performed by determining which sucrose solution was closest to the solution of derivative 2 in the sweetness intensity. The result of calculation of the average of points of 20 panelists was 2.25 point.

The sweetness intensity of the solution of derivative 2 was 8.75% according to the following equation: (10.0-8.33) x 0.25 +8.33=8.75. Therefore, the magnification of sweetness intensity of derivative 2 was 5600 (=8.75/0.00155) times that of sucrose. When the same experimentwas performed, the magnification of sweetness intensity of derivative 1 was 22600 times that of sucrose.

Furthermore, the magnification of sweetness intensity of other derivatives (3 to 9) can be determined by the same method.

And the magnification of sweetness in the cola drink can be also determined by the same method compared to the control solution of cola drink containing 10% sucrose.

The composition of cola drink is as follows.

| | |
|---|---|
| Citric acid (crystalline) | 0.25g/1000ml |
| Sodium citrate | 0.10g/1000ml |
| 85% Phosphoric aid | 0.3g/1000ml |
| Cola base | 2ml/1000ml |
| Cola essence | 1ml/1000ml |
| Sweetener (sample) | Prescribed amount |

As for the concentration of the references, the sucrose concentrations of previous (a) to (e) were used. As a result, the magnification of sweetness intensity in the cola drink of derivative 1 was 22600 times, and that of derivative 2 was 4900 times.

Hereinafter, Production Examples of the aspartyl dipeptide ester derivatives which are used for the present invention are shown.

(Production Example 1) Production of derivative 1 Synthesis of N-[N-[3-(3-hydroxy-4-methoxyphenyl) propyl] -L-α-aspartyl]-L-phenylalanine 1-methyl ester

To 485 mg (1.0 mmol) of N-t-butoxycarbonyl-β-o-benzyl -α-L-aspartyl-L-phenylalanine methyl ester, 5 ml of a 4N-HCl/dioxane solution were added and stirred at room temperature for one hour. The reaction solution was concentrated under reduced pressure. To the residue were added 30 ml of a 5%-aqueous solution of sodium hydrogen carbonate and extraction was made twice with 30 ml of ethyl acetate. An organic layer was washed with a saturated saline water and dried over anhydrous magnesium sulfate. Magnesium sulfate was filtered off and the liquid filtrate was concentrated under reduced pressure to yield 385 mg of β-o-benzyl-α-L-aspartyl-L-phenylalanine methyl ester, as a viscous oily substance.

385 mg (1.0 mmol) of the above β-o-benzyl-α-L-aspartyl -L-phenylalanine methyl ester were dissolved in 15 ml of tetrahydrofuran (THF) to yield a solution which was maintained at 0 °C. To this solution were added 268 mg (1.0 mmol) of 3-benzyloxy-4-methoxycinnamaldehyde, 0.060 ml (1.0 mmol) of acetic acid and 318 mg (1.5 mmol) of NaB $(OAc)_3H$ and stirred for one hour at 0 °C and overnight at room temperature. To the reaction solution were added 50 ml of a saturated aqueous solution of sodium hydrogen carbonate and extraction was made twice with 30 ml of ethyl acetate. An organic layer was washed with a saturated saline water and dried over anhydrous magnesium sulfate. Magnesium sulfate was filtered off and the liquid filtrate was concentrated under reduced pressure. The residue was purified with preparative thin layer chromatography (PTLC) to yield 523 mg (0.82 mmol) of N-[N-[3-(3-benzyloxy-4-methoxyphenyl) propenyl]-β-o-benzyl-L-α-aspartyl]-L-phenylalanine 1-methyl ester as a viscous oily substance. To above 523 mg (0.82 mmol) of N-[N-[3-(3-benzyloxy-4-methoxyphenyl) propenyl]-β-o-benzyl-L-α-aspartyl]-L-phenylalanine 1-methyl ester were dissolved in a mixed solvent of 30 ml of methanol and 1 ml of water, and 200 mg of 10% palladium carbon (containing 50% of water) were added thereto. The resulting mixture was reduced at room temperature for three hours under a hydrogen atmosphere. The catalyst was filtered off and the filtrate was concentrated under reduced pressure. The residue was purified with PTLC to remove an odor adsorbed to yield 228 mg (0.48 mmol) of N-[N-[3-(3-hydroxy-4-methoxyphenyl) propyl]-L-α-aspartyl]-L-phenylalanine 1-methyl ester as a solid substance.

$^1$HNMR (DMSO-$d_6$) δ:1.50-1.60 (m, 2H), 2.15-2.40 (m,6H), 2.87-2.97 (dd, 1H), 3.05-3.13 (dd, 1H), 3.37-3.43 (m, 1H), 3.62 (s, 3H), 3.71 (s, 3H), 4.50-4.60 (m,1H), 6.52 (d, 1H), 6.60 (s,1H), 6.79 (d, 1H), 7.18-7.30 (m, 5H), 8.52 (d, 1H), 8.80 (brs, 1H).

ESI(Electrospray Ionization)-MS 459.2 ($MH^+$).

(Production example 2) Production of derivative 2

Synthesis of N-[N-[3-(4-methoxyphenyl) propyl]-L-α -aspartyl]-L-phenylalanine 1-methyl ester

405 mg (2.5 mmol) of 4-methoxycinnamaldehyde, 735 mg (2.5 mmol) of aspartame and 350 mg of 10% palladium carbon (containing 50% of water) were added to a mixed solvent of 15 ml of methanol and 5 ml of water, stirred overnight at room temperature under a hydrogen atmosphere. The catalyst was filtered off and the filtrate was concentrated under reduced pressure. To the residue were added 30 ml of ethyl acetate, stirred for a while and then insoluble materials ware collected by filtration. After washing the collected insoluble materials with a little amount of ethyl acetate, 50 ml of a mixed solvent of ethyl acetate and methanol (5:2) were added to them and they were stirred for a while. Insoluble materials were removed by filtration, and the filtrate was concentrated until all the residue became the solid. This was dried under reduced pressure, and recrystalized in the mixed solvent of methanol and water, to obtain N-[N-[3-(4-methoxyphenyl) propyl]-L-α-aspartyl]-L-phenylalanine 1-methyl ester as a solid with a total yield of 43.4%.

(Production Example 3) Production of derivative 3

Synthesis of N-[N-[3-(3-hydroxy-4-methoxyphenyl)-3 -methylbutyl]-L-α-aspartyl]-L-phenylalanine 1-methyl ester

To 703 mg (1.45 mmol) of N-t-butoxycarbonyl-β-o -benzyl-(α-L-aspartyl-L-phenylalanine methylester, 10 ml of a 4N-HCl/dioxane solution were added and stirred at room temperature for one hour. The reaction solution was concentrated under reduced pressure. To the residue were added 50 ml of a 5%-aqueous solution of sodium hydrogen carbonate and extraction was made twice with 50 ml of ethyl acetate. An organic layer was washed with a saturated

saline wter and dried over anhydrous magnesium sulfate. Magnesium sulfate was filtered off and the liquid filtrate was concentrated under reduced pressure to yield 557 mg (1.45 mmo) of β-o-benzyl-α-L-aspartyl-L-phenylalanine methyl ester, as a viscous oily substance.

557 mg (1.45 mmol) of the above β-o-benzyl-α-L-aspartyl-L-phenylalanine methyl ester were dissolved in 15 ml of tetrahydrofuran (THF) to yield a solution which was maintained at 0 °C. To this solution were added 432 mg (1.45 mmol) of 3-(3-benzyloxy-4-methoxyphenyl)-3-methylbutyl aldehyde, 0.083 ml (1.45 mmol) of acetic acid and 462 mg (2.18 mmol) of $NaB(OAc)_3H$ and stirred for one hour at 0 °C and overnight at room temperature. To the reaction solution were added 50 ml of a saturated aqueous solution of sodium hydrogen carbonate and extraction was made twice with 50 ml of ethyl acetate. An organic layer was washed with a saturated saline water and dried over anhydrous magnesium sulfate. Magnesium sulfate was filtered off and the liquid filtrate was concentrated under reduced pressure. The residue was purified with preparative thin layer chromatography (PTLC) to yield 832 mg (1.25 mmol) of N-[N-[3-(3-benzyloxy-4-methoxyphenyl)-3-methylbutyl]-β-o-benzyl-L-α-aspartyl]-L-phenylalanine 1-methyl ester as a viscous oily substance. To above 832 mg (1.25 mmol) of N-[N-[3-(3-benzyloxy-4-methoxyphenyl)-3-methylbutyl]-β-o-benzyl-L-α-aspartyl]-L-phenylalanine 1-methyl ester were dissolved in a mixed solvent of 25 ml of methanol and 2 ml of water, and 350 mg of 10% palladium carbon (containing 50% of water) were added thereto. The resulting mixture was reduced at room temperature for three hours under a hydrogen atmosphere. The catalyst was filtered off and the filtrate was concentrated under reduced pressure. The residue was purified with PTLC to remove an odor adsorbed to yield 400 mg (0.82 mmol) of N-[N-[3-(3-hydroxy-4-methoxyphenyl)-3-methylbutyl]-L-α-aspartyl]-L-phenylalanine 1-methyl ester as a solid substance.

$^1$HNMR (DMSO-$d_6$) δ:1.14 (s,6H), 1.54-1.68(m,2H), 2.04-2.22 (m,3H), 2.24-2.34 (dd, 1H), 2.84-2.94 (dd, 1H), 3.00-3.08 (dd, 1H), 3.31-3.36 (m, 1H), 3.59 (s, 3H), 3.71 (s,3H), 4.46-4.55 (m, 1H), 6.60-6.65 (dd, 1H), 6.73 (s, 1H), 6.80 (d, 1H), 7.10-7.28 (m, 5H), 8.45 (d, 1H), 8.75 (brs, 1H).

ESI-MS 487.3 ($MH^+$)

(Production Example 4) Production of derivative 4 Synthesis of N-[N-[3-(3-methyl-4-hydroxyphenyl)-3-methylbutyl]-L-α-aspartyl]-L-phenylalanine 1-methyl ester

N-[N-[3-(3-methyl-4-hydroxyphenyl)-3-methylbutyl]-L-α-aspartyl]-L-phenylalanine 1-methyl ester was obtained as a solid substance, with a total yield of 63.2%, in the same way as in Production Example 3, except using 3-(3-methyl -4-benzyloxyphenyl)-3-methylbutyl aldehyde in place of 3-(3-benzyloxy-4-methoxyphenyl)-3-methylbutyl aldehyde.

$^1$HNMR (DMSO-$d_6$) δ:1.14 (s,6H), 1.59-1.68 (m,2H), 2.09 (s,3H), 2.09-2.18 (m,3H), 2.25 (dd,1H), 2.90 (dd,1H), 3.02 (dd,1H), 3.30-3.36 (m,1H), 3.59 (s,3H), 4.46-4.54 (m,1H), 6.68 (d,1H), 6.88 (dd,1H), 6.96 (s,1H), 6.14-6.73 (m, 5H), 8.46 (d,1H), 9.01 (brs, 1H).

ESI-MS 471.4 ($MH^+$)

(Production Example 5) Production of derivative 5 Synthesis of N-[N-[3-(4-methoxyphenyl)-3-methylbutyl]-L-α-aspartyl] -L-phenylalanine 1-methyl ester

N-[N-[3-(4-methoxyphenyl)-3-methylbutyl]-L-α-aspartyl]-L-phenylalanine 1-methyl ester was obtained as a solid substance, with a total yield of 72.2%, in the same way as in Production Example 3, except using 3-(4-methoxyphenyl)-3-methylbutyl aldehyde in place of 3-(3-benzyloxy-4-methoxyphenyl)-3-methylbutyl aldehyde.

$^1$HNMR (DMSO-$d_6$) δ:1.17 (s, 6H), 1.62-1.72 (m,2H); 2.04-2.20 (m, 3H), 2.24-2.34 (dd, 1H), 2:84-2.94 (dd, 1H), 2.95-3.07 (dd,1H), 3.30-3.35 (m, 1H), 3.51 (s, 3H), 3.70 (s,3H), 4.46-4.54 (m,1H), 6.83 (d,2H), 7.14-7.28 (m, 7H), 8.43 (d, 1H).

ESI-MS 471.3 ($MH^+$)

(Production Example 6) Production of derivative 6 Synthesis of N-[N-[3-(4-hydroxyphenyl)-3-methylbutyl]-L-α-aspartyl] -L-phenylalanine 1-methyl ester

N-[N-[3-(4-hydroxyphenyl)-3-methylbutyl]-L-α-aspartyl]-L-phenylalanine 1-methyl ester was obtained as a solid substance, with a total yield of 64.5%, in the same way as in Production Example 3, except using 3-(4-benzyloxyphenyl)-3-methylbutyl aldehyde in place of 3-(3-benzyloxy-4-methoxyphenyl)-3-methylbutyl aldehyde.

$^1$HNMR (DMSO-$d_6$) δ:1.15 (s, 6H), 1.58-1.72 (m,2H), 2.04-2.20 (m, 3H), 2.24-2.34 (dd, 1H), 2.85-2.94 (dd, 1H), 3.00-3.08 (dd,1H), 3.30-3.36 (m, 1H), 3.59 (s, 3H), 4.46-4.55 (m,1H), 6.67 (d,2H), 7.07 (d, 2H), 7.10-7.27 (m, 5H), 8.44 (d, 1H), 9.15 (brs,1H).

ESI-MS 457.3 ($MH^+$)

(Production Example 7) Production of derivative 7 Synthesis of N-[N-[3-(2-hydroxy-4-methoxyphenyl) propyl]-L-α-aspartyl]-L-phenylalanine 1-methyl ester

N-[N-[3-(2-hydroxy-4-methoxyphenyl) propyl]-L-α-aspartyl]-L-phenylalanine 1-methyl ester was obtained as a solid substance, with a total yield of 54.4%, in the same way as in Production Example 1, except using 2-benzyloxy-4-methoxycinnamaldehyde in place of 3-benzyloxy-4-methoxycinnamaldehyde.

$^1$HNMR (DMSO-$d_6$) δ : 1.52-1.57 (m,2H), 2.20-2.31 (m, 2H), 2.26-2.41 (m, 4H), 2.88-3.11 (m, 2H), 3.41-3.43 (m, 1H), 3.62 (s, 3H), 3.65 (s, 3H), 4.53-4.59 (m,1H), 6.28-6.36 (m, 2H), 6.88-6.90 (d,1H), 7.19-7.29 (m, 5H), 8.55 (d, 1H).

ESI-MS 459.3 ($MH^+$)

(Production Example 8) Production of derivative 8 Synthesis of N-[N-[3-(3-methyl-4-hydroxyphenyl) propyl]-L-α-aspartyl]-L-phenylalanine 1-methyl ester

N-[N-[3-(3-methyl-4-hydroxyphenyl) propyl]-L-α-aspartyl]-L-phenylalanine 1-methyl ester was obtained as a solid substance, with a total yield of 32.2%, in the same way as in Production Example 1, except using 3-methyl-4-benzyloxycinnamaldehyde in place of 3-benzyloxy-4-methoxycinnamaldehyde.

$^1$HNMR (DMSO-$d_6$) δ: 1.50-1.58 (m,2H), 2.08 (s, 3H), 2.09-2.30 (m, 2H), 2.26-2.38 (m, 4H), 2.89-3.09 (m, 2H), 3.35-3.42 (m, 1H), 3.62 (s, 3H), 4.54-4.59 (m,1H), 6.65-6.83 (m, 3H), 7.19-7.28 (m, 5H), 8.52 (d, 1H), 9.04 (brs, 1H).

ESI-MS 443.4 ($MH^+$)

(Production Example 9) Production of derivative 9 Synthesis of N-[N-[3-(2, 4-dihydroxyphenyl) propyl]-L-α-aspartyl]-L-phenylalanine 1-methyl ester

N-[N-[3-(2, 4-dihydroxyphenyl) propyl]-L-α-aspartyl]-L-phenylalanine 1-methyl ester was obtained as a solid substance, with a total yield of 42.6%, in the same way as in Production Example 1, except using 345 mg (1.0 mmol) of 3-(2,4-dibenzyloxyphenyl)-2-propenylaldehyde in place of 268 mg (1.0 mmol) of 3-benzyloxy-4-methoxycinnamaldehyde.

ESI-MS 445.3 ($MH^+$)

Effect of the Invention

According to the present invention, the sweetener composition with a high intense sweetness having a well-balanced taste of good quality, which cannot be obtained in a single use of the derivative represented by said general formula (2), particularly the general formula (1), by using at the same time therewith or mixing therewith the another sweetener with a high intense sweetness used in said present invention (the first invention) or the sugar and so on used in said present invention (the second invention) can be provided. It can be used as a sweetener and an agent for imparting sweetness for food and drink and so on. For example, it exhibits superiority in the use for cola drink such as a carbonated cola and so on, and can be applicable widely for all products which is in need of sweetness without limitation to such use.

According to another embodiment of the present invention (the third invention), it was confirmed that an effect of correcting a taste with masking effect or another effect, that is an effect of removing or suppressing a bitter taste is shown by mixing the aspartyl dipeptide ester derivative(s) (one or more) represented by said general formula (2), particularly the general formula (1), to a substance of bitter taste or a product containing it and having the bitter taste for use, and further that the effect can be maintained for a long time, and these derivatives are superior as a taste modifier. Therefore, the taste modifier of another embodiment of the present invention (the third invention) can be used as a taste modifier for food and drink in need of the taste correction and a medicine and so on. Particularly, it is preferable in view of maintenance of the effect for a long time in the form of the solution.

## Claims

1. A sweetener composition with a high intense sweetness, comprising an aspartyl dipeptide ester derivative, which may be in the salt form, represented by the following general formula (1), and an another sweetener with a high intense sweetness, wherein the quality of sweetness from said derivative is improved:

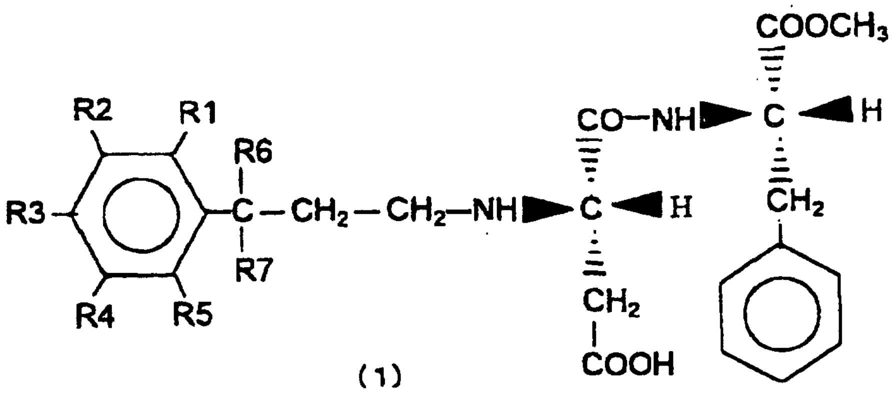

(1)

wherein $R_1$, $R_2$, $R_3$, $R_4$ and $R_5$ are independent from each other, and each denotes any one selected from the group consisting of a hydrogen atom, a hydroxyl group, a methoxy group and a methyl group, and $R_6$ and $R_7$ are independent from each other, and each denotes a hydrogen atom or a methyl group, respectively,

and when $R_6$ and $R_7$ denote different substituents each other, the carbon atom to which these substituents are linked may be in the (R), (S) or (RS) configuration.

2. The sweetener composition as defined in claim 1, wherein the sweetness intensity of said aspartyl dipeptide ester derivative is more than 4,000 times that of sucrose.

3. The sweetener composition as defined in claim 2, wherein in said general formula, $R_3$ is a hydroxyl group or a methoxy group, and $R_4$ and $R_5$ are a hydrogen atom.

4. The sweetener composition as defined in claim 3, wherein $R_1$ in said general formula is a hydroxyl group.

5. The sweetener composition as defined in claim 3, wherein $R_1$ in said general formula is a hydrogen atom.

6. The sweetener composition as defined in claim 4, wherein $R_2$, $R_6$ and $R_7$ in said general formula are a hydrogen atom.

7. The sweetener composition as defined in claim 5, wherein $R_2$ in said general formula is a hydrogen atom, a hydroxyl group or a methyl group.

8. The sweetener composition as defined in claim 1, wherein said aspartyl dipeptide ester derivative is at least one of the derivatives 1 to 9 wherein $R_1$ to $R_7$ in said general formula denote the following substituents:

| Derivative No. | $R_1$ | $R_2$ | $R_3$ | $R_4$ | $R_5$ | $R_6$ | $R_7$ |
|---|---|---|---|---|---|---|---|
| 1 | H | OH | $OCH_3$ | H | H | H | H |
| 2 | H | H | $OCH_3$ | H | H | H | H |
| 3 | H | OH | $OCH_3$ | H | H | $CH_3$ | $CH_3$ |
| 4 | H | $CH_3$ | OH | H | H | $CH_3$ | $CH_3$ |
| 5 | H | H | $OCH_3$ | H | H | $CH_3$ | $CH_3$ |
| 6 | H | H | OH | H | H | $CH_3$ | $CH_3$ |
| 7 | OH | H | $OCH_3$ | H | H | H | H |
| 8 | H | $CH_3$ | OH | H | H | H | H |
| 9 | OH | H | OH | H | H | H | H |

9. The sweetener composition as defined in claim 1, wherein said another sweetener with a high intense sweetness is at least one selected from the group consisting of Aspartame, Acesulfame K, Saccharine (including its salt form),

Sodium cyclamate, sucralose, disodium glycyrrhizinate, Alitame, Glycyrrhizin, Stevioside (including its derivative), and Thaumatin.

**10.** The sweetener composition as defined in claim 1, wherein said another sweetener with a high intense sweetness, is Aspartame.

**11.** The sweetener composition as defined in claim 10, wherein a ratio of said Aspartame to a total amount of said aspartyl dipeptide ester derivative and said Aspartame, is in the range of 5 to 90% by sweetness intensity.

**12.** The sweetener composition as defined in claim 8, wherein said aspartyl dipeptide ester derivative is the derivative No.2, said another sweetener with a high intense sweetness is Aspartame, and a ratio of said Aspartame to a total amount of said derivative No.2 and said Aspartame is in the range of 25 to 99.7% by weight.

**13.** The sweetener composition as defined in claim 1 or 2, wherein to a total amount of said aspartyl dipeptide ester derivative and said another sweetener with a high intense sweetness, a ratio of the former is in the range of 1 to 99.9% by weight.

**14.** A sweetener, food and drink, or an another sweetened product comprising the sweetener composition as defined in claim 1.

**15.** The sweetener, the food and drink, or the another sweetened product as defined in claim 14, wherein said sweetener composition is used for a drink such as a cola drink, or said food and drink is a cola drink.

**16.** A method of imparting a sweet taste comprising the step of adding or including the sweetener composition as defined in claim 1 to or in a product such as food and drink in need of a sweet taste imparted or an intermediate thereof during manufacture.

**17.** A sweetener composition with a high intense sweetness, comprising an aspartyl dipeptide ester derivative, which may be in the salt form, represented by the following general formula (1), and at least one of the compounds contained in the group consisting of sugar, sugar alcohol and oligosaccharide, wherein the quality of sweetness from said derivative is improved:

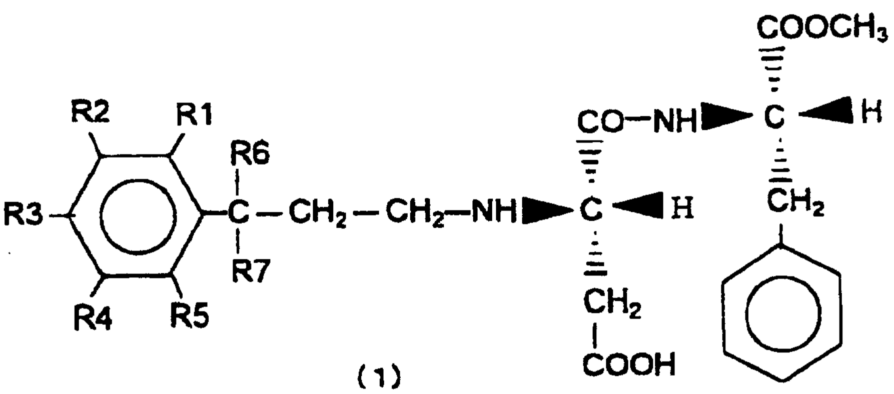

(1)

wherein $R_1$, $R_2$, $R_3$, $R_4$ and $R_5$ are independent from each other, and each denotes any one selected from the group consisting of a hydrogen atom, a hydroxyl group, a methoxy group and a methyl group, and $R_6$ and $R_7$ are independent from each other, and each denotes a hydrogen atom or a methyl group, respectively,

and when $R_6$ and $R_7$ denote different substituents each other, the carbon atom to which these substituents are linked may be in the (R), (S) or (RS) configuration.

**18.** The sweetener composition as defined in claim 17, wherein the sweetness intensity of said aspartyl dipeptide ester derivative is more than 4,000 times that of sucrose.

**19.** The sweetener composition as defined in claim 18, wherein in said general formula, $R_3$ is a hydroxyl group or a methoxy group, and $R_4$ and $R_5$ are a hydrogen atom.

**20.** The sweetener composition as defined in claim 19, wherein $R_1$ in said general formula is a hydroxyl group.

**21.** The sweetener composition as defined in claim 19, wherein $R_1$ in said general formula is a hydrogen atom.

**22.** The sweetener composition as defined in claim 20, wherein $R_2$, $R_6$ and $R_7$ in said general formula are a hydrogen atom.

**23.** The sweetener composition as defined in claim 21, wherein $R_2$ in said general formula is a hydrogen atom, a hydroxyl group or a methyl group.

**24.** The sweetener composition as defined in claim 17, wherein said aspartyl dipeptide ester derivatives is at least one of the derivatives 1 to 9 wherein $R_1$ to $R_7$ in said general formula denote the following substituents:

| Derivative No. | $R_1$ | $R_2$ | $R_3$ | $R_4$ | $R_5$ | $R_6$ | $R_7$ |
|---|---|---|---|---|---|---|---|
| 1 | H | OH | $OCH_3$ | H | H | H | H |
| 2 | H | H | $OCH_3$ | H | H | H | H |
| 3 | H | OH | $OCH_3$ | H | H | $CH_3$ | $CH_3$ |
| 4 | H | $CH_3$ | OH | H | H | $CH_3$ | $CH_3$ |
| 5 | H | H | $OCH_3$ | H | H | $CH_3$ | $CH_3$ |
| 6 | H | H | OH | H | H | $CH_3$ | $CH_3$ |
| 7 | OH | H | $OCH_3$ | H | H | H | H |
| 8 | H | $CH_3$ | OH | H | H | H | H |
| 9 | OH | H | OH | H | H | H | H |

**25.** The sweetener composition as defined in claim 17, wherein said sugar comprises sucrose (including derivative of sucrose), invert sugar, isomerized sugar, glucose, fructose, lactose, malt sugar, D-xylose and isomerized lactose; said sugar alcohol comprises maltitol, sorbitol, mannitol, erythritol, xylitol, lactitol, palatinit, and reduced starch sugar; and said oligosaccharide comprises fructooligosaccharide, maltooligosaccharide, isomaltooligosaccharide, galactooligosaccharide, soy been oligosaccharide and lactooligosaccharide.

**26.** The sweetener composition as defined in claim 17, wherein said at least one of the compounds contained in the group consisting of sugar, sugar alcohol and oligosaccharide, is sucrose, or comprises sucrose.

**27.** The sweetener composition as defined in claim 26, wherein a ratio of said sucrose to a total amount of said aspartyl dipeptide ester derivative and said sucrose is in the range of 5 to 95% by sweetness intensity.

**28.** The sweetener composition as defined in claim 17, wherein a ratio of at least one compound selected from the group consisting of sugar, sugar alcohol and oligosaccharide present, to a total amount of said aspartyl dipeptide ester derivative and said at least one compound selected from the group consisting of sugar, sugar alcohol or oligosaccharide, is in the range of 5 to 95% by sweetness intensity.

**29.** The sweetener composition as defined in claim 24, wherein said aspartyl dipeptide ester derivative is the derivative No.1, and said at least one compound selected from the group consisting of sugar, sugar alcohol or oligosaccharide, is sucrose, or comprises sucrose, and a ratio of said derivative No.1 to a total amount of said derivative No.1 and said sucrose is in the range of 5 ppm to 850 ppm by weight.

**30.** The sweetener composition as defined in claim 24, wherein said aspartyl dipeptide ester derivative is the derivative No.2, and said at least one compound selected from the group consisting of sugar, sugar alcohol and oligosaccharide, is sucrose, or comprises sucrose, and a ratio of said derivative No.2 to a total amount of said derivative No.2 and said sucrose is in the range of 6 ppm to 4000 ppm by weight.

**31.** The sweetener composition as defined in claim 17, wherein said at least one of the compounds contained in the group consisting of sugar, sugar alcohol and oligosaccharide, comprises at least one compound selected from the

group consisting of erythritol, maltitol, sorbitol and xylitol.

**32.** The sweetener composition as defined in claim 31, wherein a ratio of said sugar alcohol present, to a total amount of said aspartyl dipeptide ester derivative and said sugar alcohol, is in the range of 5 to 95% by sweetness intensity.

**33.** The sweetener composition as defined in claim 24, wherein said aspartyl dipeptide ester derivative is the derivative No.1, and a ratio of said derivative No.1 to a total amount of said derivative and said sugar alcohol, is in the range of 1 ppm to 3000 ppm by weight.

**34.** The sweetener composition as defined in claim 24, .wherein said aspartyl dipeptide ester derivative is the derivative No.2, and a ratio of said derivative No.2 to a total amount of said derivative and said sugar alcohol, is in the range of 1 ppm to 1500 ppm by weight.

**35.** The sweetener composition as defined in claim 17, wherein a ratio of said aspartyl dipeptide ester derivative to a total amount of said aspartyl dipeptide ester derivative and said sugar, sugar alcohol and oligosaccharide included, is in the range of 0.5 ppm to 5000 ppm by weight.

**36.** A sweetener, food and drink or an another sweetened product comprising the sweetener composition as defined in any one of claims 17 to 35.

**37.** The sweetener, the food and drink or the another sweetened product as defined in claim 36, wherein said sweetener composition is used for a drink such as a cola drink, or said food and drink is a cola drink.

**38.** A method of imparting a sweet taste comprising the step of adding or including the sweetener composition as defined in claim 17 to or in a product such as food and drink in need of a sweet taste imparted or an intermediate thereof during manufacture.

**39.** A taste modifier comprising an aspartyl dipeptide ester derivative, which may be in the salt form, represented by the following general formula (1):

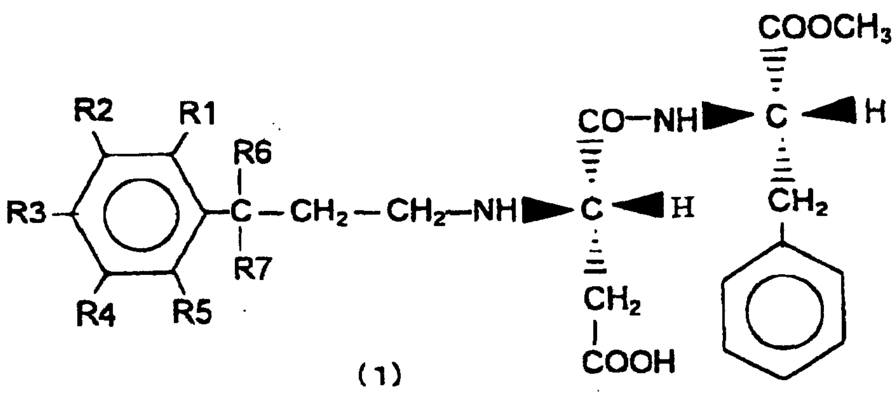

(1)

wherein $R_1$, $R_2$, $R_3$, $R_4$ and $R_5$ are independent from each other, and each denotes any one selected from the group consisting of a hydrogen atom, a hydroxyl group, a methoxy group and a methyl group, and $R_6$ and $R_7$ are independent from each other, and each denotes a hydrogen atom or a methyl group, respectively,

and when $R_6$ and $R_7$ denote different substituents each other, the carbon atom to which these substituents are linked may be in the (R), (S) or (RS) configuration.

**40.** The taste modifier as defined in claim 39, wherein the intensity of sweetness of said aspartyl dipeptide ester derivative is more than 4,000 times that of sucrose.

**41.** The taste modifier as defined in claim 40, wherein in said general formula, $R_3$ is a hydroxyl group or a methoxy group, and $R_4$ and $R_5$ are a hydrogen atom.

**42.** The taste modifier as defined in claim 41, wherein $R_1$ in said general formula is a hydroxyl group.

**43.** The taste modifier as defined in claim 41, wherein $R_1$ in said general formula is a hydrogen atom.

**44.** The taste modifier as defined in claim 42, wherein $R_2$, $R_6$ and $R_7$ in said general formula are a hydrogen atom.

**45.** The taste modifier as defined in claim 43, wherein $R_2$ in said general formula is a hydrogen atom, a hydroxyl group or a methyl group.

**46.** The taste modifier as defined in claim 39, wherein said aspartyl dipeptide ester derivatives is at least one of the derivatives 1 to 9 wherein $R_1$ to $R_7$ in said general formula denote the following substituents:

| Derivative No. | $R_1$ | $R_2$ | $R_3$ | $R_4$ | $R_5$ | $R_6$ | $R_7$ |
|---|---|---|---|---|---|---|---|
| 1 | H | OH | $OCH_3$ | H | H | H | H |
| 2 | H | H | $OCH_3$ | H | H | H | H |
| 3 | H | OH | $OCH_3$ | H | H | $CH_3$ | $CH_3$ |
| 4 | H | $CH_3$ | OH | H | H | $CH_3$ | $CH_3$ |
| 5 | H | H | $OCH_3$ | H | H | $CH_3$ | $CH_3$ |
| 6 | H | H | OH | H | H | $CH_3$ | $CH_3$ |
| 7 | OH | H | $OCH_3$ | H | H | H | H |
| 8 | H | $CH_3$ | OH | H | H | H | H |
| 9 | OH | H | OH | H | H | H | H |

**47.** Food and drink, a medicine or so on with improved bitter taste, comprising the taste modifier as defined in claim 39.

**48.** The food and drink, the medicine or so on as defined in claim 47, comprising at least one compound with said bitter taste selected from the group consisting of an amino acid, a peptide, quinine, caffeine and a mineral.

**49.** The food and drink, the medicine or so on as defined in claim 47 or 48, comprising said aspartyl dipeptide ester derivative in the range of 0.2 ppm to 10000 ppm by weight.

**50.** The food and drink, the medicine or so on as defined in claim 47, being in the form of liquid.

**51.** A method of correcting a taste comprising the step of mixing or including the taste modifier as defined in claim 39 into a product such as food and drink, which needs a correction of taste or an intermediate thereof during manufacture.

## INTERNATIONAL SEARCH REPORT

International application No.
PCT/JP00/06628

A. CLASSIFICATION OF SUBJECT MATTER
Int.Cl[7] C07K5/075, A23L1/236, A23L1/22, A23L2/60

According to International Patent Classification (IPC) or to both national classification and IPC

B. FIELDS SEARCHED

Minimum documentation searched (classification system followed by classification symbols)
Int.Cl[7] C07K5/075, A23L1/236, A23L1/22, A23L2/60

Documentation searched other than minimum documentation to the extent that such documents are included in the fields searched

Electronic data base consulted during the international search (name of data base and, where practicable, search terms used)
CA(STN),REGISTRY(STN),WPI(DIALOG)

C. DOCUMENTS CONSIDERED TO BE RELEVANT

| Category* | Citation of document, with indication, where appropriate, of the relevant passages | Relevant to claim No. |
|---|---|---|
| X/A | WO, 9411391, A1 (Nofre Cloude), 26 May, 1994 (26.05.94) & US, 5480668, A & EP, 669935, A1 & JP, 8-503206, T2 | 1-38/ 39-51 |
| A | WO, 00/00508, A1 (Ajinomoto Co., Inc.), 07 June, 1999 (07.06.99) & AU, 9940602, A | 1-51 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 December, 2000 (19.12.00) | 26 December, 2000 (26.12.00) |
| Name and mailing address of the ISA/ Japanese Patent Office | Authorized officer |
| Facsimile No. | Telephone No. |

Form PCT/ISA/210 (second sheet) (July 1992)